# EUROPEAN PATENT APPLICATION

(11) **EP 4 324 558 A1**
(43) Date of publication of application: **21.02.2024**
(21) Application number: 22787881.6
(22) Date of filing: 02.03.2022
(51) Int. Cl.: B01J 35/04, B01J 35/10, C07B 37/04, C07C 22/08, C07C 201/14, C07C 205/06, C07C 253/30, C07C 255/50, C07C 17/26, C07B 61/00, C08J 5/20, B01J 41/05, B01J 41/14, B01J 47/12, B01J 31/08

(54) **ION EXCHANGER, METHOD FOR PRODUCING ION EXCHANGER, CATALYST HAVING PLATINUM-GROUP MEAL ION SUPPORTED THEREON, AND METHOD FOR FORMING CARBON-CARBON BOND**

(30) Priority: 14.04.2021 JP 2021068532; 14.04.2021 JP 2021068533
(71) Applicant: Organo Corporation, Tokyo 136-8631 (JP)
(72) Inventor: NAKAMURA, Shinji, Tokyo 136-8631 (JP); SAJIKI, Hironao, Gifu-shi, Gifu 501-1196 (JP); YAMADA, Tsuyoshi, Gifu-shi, Gifu 501-1196 (JP); PARK, Kwihwan, Gifu-shi, Gifu 501-1196 (JP); YAMADA, Yutaro, Gifu-shi, Gifu 501-1196 (JP)
(74) Representative: Müller-Boré & Partner Patentanwälte PartG mbB
(86) International application number: PCT/JP2022/008803
(87) International publication number: WO 2022/219953

(57) **Abstract**

The purpose of the present invention is to provide: an ion exchanger which shows small shrinkage upon the contact with an organic solvent in a water-wetted state; and a method for producing the ion exchanger. Provided is an ion exchanger which is composed of a polymer chain represented by general formula (1) 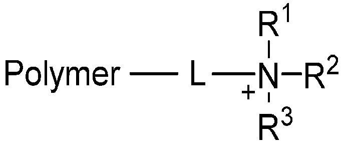 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; R² and R³ each independently represent an alkyl group of 1 to 4 carbon atoms; L represents a linker site; and "Polymer" represents a polymer chain).

## Description

### TECHNICAL FIELD

The present invention relates to an ion exchanger, a method for producing an ion exchanger, a catalyst having a platinum group metal ion supported thereon, and a method for forming a carbon-carbon bond by conducting a reaction that generates a carbon-carbon bond using the catalyst having a platinum group metal ion supported thereon.

### BACKGROUND

Ion exchange reactions are important in the purification of water and sugar solutions and the like, and in synthetic processes and the like for chemical products such as pharmaceutical intermediates, and ion exchangers including ion exchange resins are widely used. In recent years, as higher purity levels are being demanded for organic solvents, methods for purifying organic solvents using ion exchangers have also been developed, and for example, Patent Document 1 discloses a method for purifying an organic solvent such as 2-propanol containing multivalent metal ions by bringing the organic solvent into contact with a column packed with a monolithic organic porous ion exchanger.

However, investigations by the inventors of the present invention revealed that when an organic solvent is passed through a column packed with a monolithic organic porous ion exchanger in a water-wet state, the monolithic organic porous ion exchanger shrinks dramatically, raising a problem in that the organic solvent does not efficiently contact the monolithic organic porous ion exchanger.

On the other hand, carbon-carbon formation reactions (coupling reactions) that use a platinum group metal such as palladium as a catalyst, typified by Suzuki-Miyaura coupling, Sonogashira coupling and Mizoroki-Heck coupling, have in recent years continued to grow in importance in synthetic processes and the like for pharmaceutical intermediates and high-performance materials such as organic EL materials and the like.

Conventionally, the platinum group metal catalysts mentioned above have often been used in homogenous systems, and exhibit high levels of catalytic activity, but problems have included difficulties in recovering the catalyst and contamination of the product with the metal of the catalyst. Accordingly, heterogeneous catalysts in which a catalyst mentioned above is supported on a carrier have been developed, and these catalysts enable easier recovery of the catalyst and reduced metal contamination of the product, and in recent years, methods for continuously forming carbon-carbon bonds by passing a reactant solution across a heterogeneous catalyst have also been developed.

For example, Patent Document 2 reports a method for continuously forming carbon-carbon bonds using a catalyst composed of palladium supported on a porous silica carrier, but no actual reaction examples are disclosed.

Patent Document 3 and Patent Document 4 disclose methods for forming carbon-carbon bonds using a catalyst composed of a platinum group metal supported on the wall surface of a fine flow passage with a width of 1 mm and a depth of 20 µm, but for unknown reasons, the disclosed reaction examples do not use water as a solvent, and tests were only conducted at a reactant solution flow rate of 1 µm/minute, and therefore the environmental load and production efficiency are problematic.

Non-Patent Document 1 reports a method for continuously forming carbon-carbon bonds using a column packed with a catalyst composed of palladium supported on an organic carrier, but a complex chemical conversion is required to obtain the organic carrier, and although the reasons are unclear, when a catalyst on which palladium has been supported is used, a large amount of diatomaceous earth must be mixed with the carrier.

On the other hand, the inventors of the present invention have developed catalysts having a platinum group metal supported on a non-particulate organic ion exchanger having three-dimensionally continuous pores, and in Patent Document 5 and Patent Document 6, have reported that these catalysts exhibit high catalytic activity in aqueous solvents for carbon-carbon bond forming reactions.

According to the methods disclosed in Patent Document 5 and Patent Document 6, by using a platinum group metal supported catalyst having a platinum group metal supported on a non-particulate organic ion exchanger as a catalyst, carbon-carbon bond forming reactions can be conducted in aqueous solvents with high yield.

However, in the reaction examples of Patent Document 5 and Patent Document 6, a carbon-carbon bond forming reaction was able to be conducted with a high yield only when the organic halide raw material was a highly reactive aromatic iodide, and in the case of aromatic bromides, the yield was unsatisfactory, meaning the number of raw materials that can be used is limited.

### CITATION LIST

### PATENT LITERATURE

Patent Document 1: JP 2017-119233 A
Patent Document 2: JP 5638862 B
Patent Document 3: JP 5255215 B
Patent Document 4: JP 2008-212765 A
Patent Document 5: JP 2014-015420 A
Patent Document 6: JP 2016-190853 A

### NON-PATENT LITERATURE

Non-Patent Document 1: ChemCatChem 2019, Vol. 11, p. 2427

### SUMMARY

### TECHNICAL PROBLEM

An object of the present invention is to provide an ion exchanger that exhibits little shrinkage upon contact in a water-wet state with an organic solvent, and a method for producing that ion exchanger.

Further, another object of the present invention is to provide a catalyst with a platinum group metal supported thereon that enables a carbon-carbon bond forming reaction to be conducted with high yield even with an aromatic bromide, and a method for forming a carbon-carbon bond using that catalyst with a platinum group metal supported thereon.

### SOLUTION TO PROBLEM

The present invention provides an ion exchanger composed of a polymer chain represented by general formula (1): 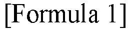 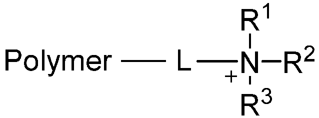 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; R² and R³ each independently represent an alkyl group of 1 to 4 carbon atoms; L represents a linker site; and "Polymer" represents a polymer chain).

In the ion exchanger described above, L in general formula (1) is preferably a methylene group.

In the ion exchanger described above, the polymer chain of the ion exchanger is preferably a styrene-divinylbenzene-based copolymer.

In the ion exchanger described above, it is preferable that the ion exchanger is a non-particulate organic porous ion exchanger composed of a continuous skeleton phase and a continuous pore phase, wherein the thickness of the continuous skeleton is within a range from 1 to 100 µm, the average diameter of the continuous pores is within a range from 1 to 1,000 µm, the total pore volume is within a range from 0.5 to 50 mL/g, the ion exchange capacity per unit weight in a dry state is within a range from 1 to 9 mg equivalent/g, and the ion exchange groups are distributed throughout the organic porous ion exchanger.

The present invention also provides a method for producing an ion exchanger by reacting:
a polymer chain represented by general formula (2):

   [Formula 2] Polymer-L-X
(wherein L represents a linker site, "Polymer" represents a polymer chain, and X represents a halogen atom, an alkylsulfonyl group of 1 to 8 carbon atoms which may be substituted, or a benzenesulfonyl group which may be substituted), and
a tertiary amine represented by general formula (3): 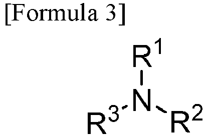 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; and R² and R³ each independently represent an alkyl group of 1 to 4 carbon atoms).

In the method for producing an ion exchanger described above, L in general formula (1) is preferably a methylene group.

In the method for producing an ion exchanger described above, the polymer chain of the ion exchanger is preferably a styrene-divinylbenzene-based copolymer.

In the method for producing an ion exchanger described above, it is preferable that the ion exchanger is a non-particulate organic porous ion exchanger composed of a continuous skeleton phase and a continuous pore phase, wherein the thickness of the continuous skeleton is within a range from 1 to 100 µm, the average diameter of the continuous pores is within a range from 1 to 1,000 µm, the total pore volume is within a range from 0.5 to 50 mL/g, the ion exchange capacity per unit weight in a dry state is within a range from 1 to 9 mg equivalent/g, and the ion exchange groups are distributed throughout the organic porous ion exchanger.

The present invention also provides a catalyst having a platinum group metal ion supported thereon, wherein at least one of a platinum group metal ion and a platinum group metal complex ion are supported on the ion exchanger described above.

In the catalyst having a platinum group metal ion supported thereon described above, it is preferable that the non-particulate organic porous ion exchanger has a continuous macroporous structure having common openings with an average diameter within a range from 1 to 1,000 µm formed in the walls of mutually joined macropores, wherein the total pore volume is within a range from 1 to 50 mL/g, the ion exchange capacity per unit weight in a dry state is within a range from 1 to 9 mg equivalent/g, and the ion exchange groups are distributed throughout the organic porous ion exchanger.

In the catalyst having a platinum group metal ion supported thereon described above, it is preferable that the non-particulate organic porous ion exchanger forms a three-dimensionally continuous skeleton portion composed of organic polymer particles with an average particle diameter within a range from 1 to 50 µm aggregated together, and has three-dimensionally continuous pores with an average diameter within a range from 20 to 100 µm within that skeleton, wherein the total pore volume is within a range from 1 to 10 mL/g, the ion exchange capacity per unit weight in a dry state is within a range from 1 to 9 mg equivalent/g, and the ion exchange groups are distributed throughout the organic porous ion exchanger.

In the catalyst having a platinum group metal ion supported thereon described above, it is preferable that the non-particulate organic porous ion exchanger is a continuous macroporous structure in which bubble-shaped macropores overlap with each other, with these overlapping portions forming openings having an average diameter within a range from 30 to 300 µm, wherein the total pore volume is within a range from 0.5 to 10 mL/g, the ion exchange capacity per unit weight in a dry state is within a range from 1 to 9 mg equivalent/g, the ion exchange groups are distributed throughout the organic porous ion exchanger, and the surface area of the skeleton portion shown in cross-section, in an SEM image of a cross-section of the continuous macroporous structure, is within a range from 25 to 50% within the image region.

In the catalyst having a platinum group metal ion supported thereon described above, it is preferable that the non-particulate organic porous ion exchanger is a co-continuous structure composed of a three-dimensionally continuous skeleton in which the continuous skeleton, formed from an aromatic vinyl polymer containing from 0.1 to 5.0 mol% of crosslinked structural units among all of the structural units having an introduced ion exchange group, has a thickness within a range from 1 to 60 µm, and three-dimensionally continuous pores with an average diameter within a range from 10 to 200 µm within that skeleton, wherein the total pore volume is within a range from 0.5 to 10 mL/g, the ion exchange capacity per unit weight in a dry state is within a range from 1 to 9 mg equivalent/g, the ion exchange groups are distributed throughout the organic porous ion exchanger.

In the catalyst having a platinum group metal ion supported thereon described above, it is preferable that the non-particulate organic porous ion exchanger is composed of a continuous skeleton phase and a continuous pore phase, wherein the skeleton has a plurality of particle bodies with a diameter within a range from 4 to 40 µm adhered to the skeleton surface or a plurality of protrusions with a size within a range from 4 to 40 µm formed on the surface of the skeleton of the organic porous body, the average diameter of the continuous pores is within a range from 10 to 200 µm, the total pore volume is within a range from 0.5 to 10 mL/g, the ion exchange capacity per unit weight in a dry state is within a range from 1 to 9 mg equivalent/g, and the ion exchange groups are distributed throughout the organic porous ion exchanger.

In the catalyst having a platinum group metal ion supported thereon described above, the amount supported of at least one of the platinum group metal ion and the platinum group metal complex ion, expressed as an equivalent mass of the platinum group metal atoms, is preferably within a range from 0.01 to 10.0% by mass.

In the catalyst having a platinum group metal ion supported thereon described above, R¹ in general formula (1) is preferably an alkyl group of 6 to 20 carbon atoms, or a benzyl group which may be substituted with an alkyl group of 1 to 4 carbon atoms.

In the catalyst having a platinum group metal ion supported thereon described above, R¹ in general formula (1) is preferably a dodecyl group or a benzyl group.

In the catalyst having a platinum group metal ion supported thereon described above, R² and R³ in general formula (1) preferably each independently represent a methyl group or an ethyl group.

In the catalyst having a platinum group metal ion supported thereon described above, R² and R³ in general formula (1) are preferably both methyl groups.

The present invention also provides a method for forming a carbon-carbon bond in which a carbon-carbon bond is formed by conducting (1) a reaction between an aromatic halide and an organic boron compound, (2) a reaction between an aromatic halide and a compound having an alkynyl group at a terminal, or (3) a reaction between an aromatic halide and a compound having an alkenyl group, wherein the formation reaction for the carbon-carbon bond is conducted by introducing a raw material liquid (i) containing the aromatic halide and the organic boron compound, a raw material liquid (ii) containing the aromatic halide and the compound having an alkynyl group at a terminal, or a raw material liquid (iii) containing the aromatic halide and the compound having an alkenyl group into a packed container packed with a catalyst having a platinum group metal ion supported thereon from an introduction passage of the packed container, passing the raw material liquid through the catalyst having a platinum group metal ion supported thereon, and discharging the reaction liquid from a discharge passage of the packed container, wherein the catalyst having a platinum group metal ion supported thereon is the catalyst having a platinum group metal ion supported thereon according to any one of Claims 1 to 8.

In the method for forming a carbon-carbon bond described above, the formation reaction for the carbon-carbon bond is preferably conducted in the presence of an inorganic base.

In the method for forming a carbon-carbon bond described above, it is preferable that the raw material liquid (i), the raw material liquid (ii) or the raw material liquid (iii) is an inorganic base-dissolved raw material liquid prepared by dissolving the raw materials and an inorganic base in water or a hydrophilic solvent, and that the formation reaction for the carbon-carbon bond is conducted by introducing the inorganic base-dissolved raw material liquid into the packed container packed with the catalyst having a platinum group metal ion supported thereon from an introduction passage of the packed container, passing the inorganic base-dissolved raw material liquid through the catalyst having a platinum group metal ion supported thereon, and discharging the reaction liquid from a discharge passage of the packed container.

In the method for forming a carbon-carbon bond described above, it is preferable that the raw material liquid (i), the raw material liquid (ii) or the raw material liquid (iii) is a hydrophobic solvent raw material liquid prepared by dissolving the raw materials in a hydrophobic organic solvent, and that the formation reaction for the carbon-carbon bond is conducted by introducing a mixture of the hydrophobic solvent raw material liquid and an inorganic base aqueous solution prepared by dissolving an inorganic base into the packed container packed with the catalyst having a platinum group metal ion supported thereon from an introduction passage of the packed container, passing the hydrophobic solvent raw material liquid and the inorganic base aqueous solution through the catalyst having a platinum group metal ion supported thereon, and discharging the reaction liquid from a discharge passage of the packed container.

### ADVANTAGEOUS EFFECTS OF INVENTION

The present invention is able to provide an ion exchanger that exhibits little shrinkage upon contact in a water-wet state with an organic solvent, and a method for producing that ion exchanger.

Further, the present invention is also able to provide a catalyst with a platinum group metal ion supported thereon that enables a carbon-carbon bond forming reaction to be conducted with high yield even with an aromatic bromide, and a method for forming a carbon-carbon bond using that catalyst with a platinum group metal ion supported thereon.

### BRIEF DESCRIPTION OF DRAWINGS

FIG. 1 is a SEM photograph of an example of the morphology of a monolith No. 1.
FIG. 2 is a SEM photograph of an example of the morphology of a monolith No. 2.
FIG. 3 is a SEM photograph of an example of the morphology of a monolith No. 3.
FIG. 4 is a diagram illustrating the skeleton portion represented as a cross-section of the SEM photograph of FIG. 3.
FIG. 5 is a SEM photograph of an example of the morphology of a monolith No. 4.
FIG. 6 is a schematic illustration of the co-continuous structure of the monolith No. 4 and a weakly basic monolithic ion exchanger.
FIG. 7 is a SEM photograph of an example of the morphology of a monolith intermediate (4).
FIG. 8 is a schematic cross-sectional view of protrusions.
FIG. 9 is a SEM photograph of an example of the morphology of a monolith No. 5-1.
FIG. 10 is a SEM photograph of a monolith intermediate obtained in Example 1.
FIG. 11 is a SEM photograph of a monolith obtained in Example 1.
FIG. 12 is a graph illustrating the swelling ratio (%) (length ratio) when water-wet state monolithic anion exchangers are brought into contact with various organic solvents in Example 2 and Comparative Example 2.

### DESCRIPTION OF EMBODIMENTS

Embodiments of the present invention are described below. These embodiments are merely examples of implementing the present invention, and the present invention is not limited to these embodiments.

### <Ion Exchanger>

The ion exchanger according to an embodiment of the present invention is composed of a polymer chain represented by general formula (1): 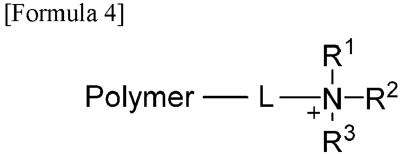 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; R² and R³ each independently represent an alkyl group of 1 to 4 carbon atoms; L represents a linker site; and "Polymer" represents a polymer chain).

The inventors of the present invention discovered that an ion exchanger composed of a polymer chain represented by the above general formula (1) exhibits little shrinkage upon contact in a water-wet state with an organic solvent. Even when this ion exchanger is packed in a column in a water-wet state and an organic solvent is then passed through the column, the ion exchanger undergoes almost no shrinkage, and the organic solvent is able to efficiently contact the ion exchanger. It is thought that this is because the hydrophobicity is increased as a result of the ion exchanger having an alkyl group of 4 to 22 carbon atoms or a benzyl group as R¹.

By using the ion exchanger according to this embodiment, the shrinkage rate upon contact in a water-wet state with an organic solvent is, for example, can be kept within ±10% of the value prior to contact with the organic solvent, and preferably within ±8%. There are no particular restrictions on the organic solvent, and examples include alcohols such as methanol and 2-propanol, amide-based solvents such as N,N-dimethylformamide (DMF) and N-methyl-2-pyrrolidone (NMP), ether-based solvents such as diisopropyl ether and tetrahydrofuran (THF), aromatic-based solvents such as toluene and xylene, and hydrocarbon-based solvents such as pentane and hexane.

R¹ in general formula (1) represents an alkyl group of 4 to 22 carbon atoms which may be substituted. Further, R¹ also represents a benzyl group which may be substituted with an alkyl group of 1 to 6 carbon atoms which may be substituted, a benzyl group which may be substituted with a halogen atom, a benzyl group which may be substituted with an alkoxy group of 1 to 6 carbon atoms which may be substituted, a benzyl group which may be substituted with an amino group which may be substituted, a benzyl group which may be substituted with a cyano group, or a benzyl group which may be substituted with a nitro group. The benzyl group which may be substituted is a benzyl group in which the phenyl group of the benzyl group may be substituted with a substituent.

The alkyl group of 4 to 22 carbon atoms may be linear, branched or cyclic. In terms of factors such as ensuring a high reaction yield during production, ease of availability of the raw material, and more favorable performance, an alkyl group of 6 to 20 carbon atoms is preferred, and an alkyl group of 6 to 18 carbon atoms is more preferred.

The alkyl group of 1 to 6 carbon atoms with which the benzyl group may be substituted may be linear, branched or cyclic. In terms of factors such as ensuring ease of availability of the raw material and more favorable performance, an alkyl group of 1 to 4 carbon atoms is preferred, and a methyl group or an ethyl group is more preferred.

Examples of the halogen atom with which the benzyl group may be substituted include a chlorine atom, a bromine atom and an iodine atom, and in terms of ensuring inexpensive raw material production and ease of availability of the raw material, a chlorine atom is preferred.

The alkoxy group of 1 to 6 carbon atoms with which the benzyl group may be substituted may be linear, branched or cyclic. In terms of factors such as ensuring ease of availability of the raw material and more favorable performance, an alkoxy group of 1 to 4 carbon atoms is preferred, and a methoxy group or ethoxy group is more preferred.

Examples of the substituent in those cases where the alkyl group of 4 to 22 carbon atoms, the alkyl group of 1 to 6 carbon atoms, or the alkoxy group of 1 to 6 carbon atoms "may be substituted" include a nitro group, a cyano group, and a halogen atom.

Examples of the substituent in those cases where the amino group "may be substituted" include an alkyl group of 1 to 8 carbon atoms, a phenyl group, and a fluoroalkyl group of 1 to 4 carbon atoms.

The alkyl group of 1 to 8 carbon atoms with which the amino group may be substituted may be linear, branched or cyclic. In terms of factors such as ensuring a high reaction yield during production, an alkyl group of 1 to 4 carbon atoms is preferred, and a methyl group or an ethyl group is more preferred.

The fluoroalkyl group of 1 to 4 carbon atoms with which the amino group may be substituted may be linear, branched or cyclic. In terms of factors such as ensuring ease of availability of the raw material, a fluoroalkyl group of 1 or 2 carbon atoms is preferred, and a trifluoromethyl group or a 2,2,2-trifluoroethyl group is more preferred.

R¹ in general formula (1) is preferably an alkyl group of 6 to 20 carbon atoms or a benzyl group which may be substituted with an alkyl group of 1 to 4 carbon atoms, and is more preferably a dodecyl group or a benzyl group.

R² and R³ in general formula (1) each independently represent an alkyl group of 1 to 4 carbon atoms. The alkyl group of 1 to 4 carbon atoms may be linear, branched or cyclic. In terms of factors such as ensuring a high reaction yield during production, a methyl group or an ethyl group is preferred, and a methyl group is more preferred.

L in general formula (1) represents a linker site. Examples of the linker site include an alkylene group of 1 to 4 carbon atoms which may be substituted with an oxy group. In terms of factors such as ensuring a high reaction yield during production, a methylene group, ethylene group or 1-oxyethylene group is preferred, and a methylene group is more preferred.

In general formula (1), "Polymer" represents a polymer chain. Examples of the polymer chain include crosslinked polymers including aromatic vinyl polymers such as polystyrene, poly(α-methylstyrene) and polyvinylbenzyl chloride; polyolefins such as polyethylene and polypropylene; poly(halogenated polyolefins) such as polyvinyl chloride and polytetrafluoroethylene; nitrile-based polymers such as polyacrylonitrile; and (meth)acrylic-based polymers such as polymethacrylate esters and polyacrylate esters. The polymer chain may be a polymer obtained by copolymerizing a single vinyl monomer and a crosslinking agent, a polymer obtained by polymerizing two or more vinyl monomers and a crosslinking agent, or a blend of two or more polymers. Among these, in terms of the ease of formation of the continuous structure, the ease of introduction of the ion exchange groups, the mechanical strength, and the stability relative to acid or alkali, a crosslinked polymer of an aromatic vinyl polymer is preferred, and in particular, a styrene-divinylbenzene-based copolymer or a vinylbenzyl chloride-divinylbenzene-based copolymer is preferred, and a styrene-divinylbenzene-based copolymer is preferred.

The ion exchanger according to an embodiment of the present invention may be a particulate ion exchanger or a non-particulate ion exchanger, and is preferably a non-particulate organic porous ion exchanger.

### <Catalyst having a Platinum group Metal Ion Supported thereon>

A catalyst having a platinum group metal ion supported thereon according to an embodiment of the present invention is a catalyst in which at least one of a platinum group metal ion and a platinum group metal complex ion is supported on an ion exchanger composed of a polymer chain represented by the above general formula (1).

As a result of intensive investigations, the inventors of the present invention discovered that a catalyst in which at least one of a platinum group metal ion and a platinum group metal complex ion is supported on an ion exchanger composed of a polymer chain represented by the above general formula (1), namely, a catalyst in which a platinum group metal is supported in an ionic state on an ion exchanger composed of a polymer chain represented by the above general formula (1), enables a carbon-carbon bond forming reaction to be conducted with high yield even with an aromatic bromide. In particular, the inventors discovered that the yield increased in fixed-bed continuous flow carbon-carbon bond forming reactions, even in those cases where an aromatic bromide was used as a raw material.

It is thought that these observations are due to the fact that because the ion exchanger composed of the polymer chain represented by the above general formula (1) has the aforementioned alkyl group of 4 to 22 carbon atoms or the aforementioned benzyl group as R¹, the lipophilicity increases, and in a fixed-bed continuous flow carbon-carbon bond forming reaction, the contact between the two phases, namely the organic phase and the water phase, is able to occur efficiently on the surfaces of the ion exchanger.

The ion exchanger according to an embodiment of the present invention may be either a particulate ion exchanger or a non-particulate ion exchanger, but in terms of factors such as increasing the liquid contact frequency with the raw material liquid in the carbon-carbon bond forming reaction, a non-particulate organic porous ion exchanger is preferred.

### <Non-Particulate Organic Porous Ion Exchanger>

A non-particulate organic porous ion exchanger is a substance in which ion exchange groups have been introduced into a monolithic organic porous body having a continuous skeleton phase and a continuous pore phase. The non-particulate organic porous ion exchanger composed of a polymer chain represented by the above general formula (1) is a basic monolithic organic porous anion exchanger. The monolithic organic porous body has a plurality of continuous pores that function as passages through the skeleton. In this description, a "monolithic organic porous body" is also referred to as simply a "monolith", a "monolithic organic porous ion exchanger" is also referred to as simply a "monolithic ion exchanger", and a "monolithic organic porous intermediate" which represents an intermediate (precursor) in the production of a monolith is also referred to as simply a "monolith intermediate".

The structures of these non-particulate organic porous ion exchangers are disclosed in JP 2002-306976 A, JP 2009-007550 A, JP 2009-062512 A, JP 2009-067982 A and JP 2009-108294 A.

The non-particulate organic porous ion exchanger is preferably composed of a continuous skeleton phase and a continuous pore phase, wherein the thickness of the continuous skeleton is within a range from 1 to 100 µm, the average diameter of the continuous pores is within a range from 1 to 1,000 µm, the total pore volume is within a range from 0.5 to 50 mL/g, the ion exchange capacity per unit weight in a dry state is within a range from 1 to 9 mg equivalent/g, and the ion exchange groups are distributed throughout the organic porous ion exchanger. The continuous skeleton phase and the continuous pore phase can be observed using SEM images.

The thickness of the continuous skeleton of the non-particulate organic porous ion exchanger in a dry state is preferably within a range from 1 to 100 µm. The thickness of the continuous skeleton of the non-particulate organic porous ion exchanger in a dry state is determined by SEM observation. If this thickness of the continuous skeleton is less than 1 µm, then in those cases where the ion exchange capacity per unit volume decreases, or in those cases where the mechanical strength decreases and the ion exchanger is packed into a column and a treatment target liquid is then passed through the column, the non-particulate organic porous ion exchanger may sometimes deform, particularly when the liquid is passed through at a high flow rate. If this thickness of the continuous skeleton exceeds 100 µm, then the skeleton becomes too thick, and the pressure loss during liquid passage may sometimes increase.

The average diameter of the continuous pores of the non-particulate organic porous ion exchanger in a dry state is preferably within a range from 1 to 1,000 µm. The average diameter of the continuous pores of the non-particulate organic porous ion exchanger in a dry state can be measured by mercury porosimetry, and indicates the value at the maximum of the pore distribution curve obtained by mercury porosimetry. If this average diameter of the continuous pores is less than 1 µm, then when the ion exchanger is packed into a column and a treatment target liquid is passed through the column, the pressure loss during liquid passage may sometimes increase. If this average diameter of the continuous pores exceeds 1,000 µm, then when the ion exchanger is packed into a column and a treatment target liquid is passed through the column, the contact between the treatment target liquid and the ion exchanger may be unsatisfactory, causing a deterioration in the ion exchange performance.

The total pore volume of the non-particulate organic porous ion exchanger in a dry state is preferably within a range from 0.5 to 50 mL/g. The total pore volume of the non-particulate organic porous ion exchanger in a dry state can be measured by mercury porosimetry. If this total pore volume is less than 0.5 mL/g, then when the ion exchanger is packed into a column and a treatment target liquid is passed through the column, the pressure loss during liquid passage may sometimes increase. If this total pore volume exceeds 50 mL/g, then the mechanical strength of the non-particulate organic porous ion exchanger deteriorates, and when the ion exchanger is packed into a column and a treatment target liquid is passed through the column, the ion exchanger may sometimes deform, particularly when the liquid is passed through at a high flow rate, and the pressure loss during liquid passage may sometimes increase.

The ion exchange capacity per unit weight of the non-particulate organic porous ion exchanger in a dry state is preferably within a range from 1 to 9 mg equivalent/g. The ion exchange capacity per unit weight of the non-particulate organic porous ion exchanger in a dry state can be measured using a method such as neutralization titration. If this ion exchange capacity is less than 1 mg equivalent/g, then the amount of ions that can be exchanged and supported may sometimes decrease. If this ion exchange capacity exceeds 9 mg equivalent/g, then the reaction for introducing the ion exchange groups must be conducted under severe conditions, which may sometimes result in marked oxidative degradation of the monolith.

In the non-particulate organic porous ion exchanger, the introduced ion exchange groups are preferably distributed not only across the surface of the monolith, but also through the interior of the monolith skeleton, namely, throughout the organic porous ion exchanger, and are more preferably distributed uniformly. The expression that the "ion exchange groups are distributed uniformly throughout the organic porous ion exchanger" indicates that the ion exchange groups are distributed across the surface and throughout the skeleton interior of the organic porous ion exchanger down to at least the µm order. The state of the distribution of the ion exchange groups can be confirmed using an electron probe microanalyzer (EPMA). By distributing the ion exchange groups not only across the surface of the monolith, but also through the interior of the monolith skeleton, the physical properties and chemical properties of the surface and the interior of the monolith can be made almost uniform, meaning the durability relative to swelling and shrinking is improved.

In the non-particulate organic porous ion exchanger, the material that constitutes the continuous skeleton is an organic polymer material having a crosslinked structure, and is represented by the Polymer portion in the above general formula (1). Relative to the total of all the structural units that constitute the polymer material, the polymer preferably includes 0.1 to 30 mol% of crosslinked structural units, and more preferably includes 0.1 to 20 mol% of crosslinked structural units.

### <Monolithic Ion Exchangers No. 1 to No. 5>

A monolithic organic porous ion exchanger (monolithic ion exchanger) No. 1 through to a monolithic organic porous ion exchanger (monolithic ion exchanger) No. 5 are described below as examples of more specific embodiments of the non-particulate organic porous ion exchanger. In the following description, descriptions are omitted for those structures that are the same as in the non-particulate organic porous ion exchangers described above.

### <Monolithic Ion Exchanger No. 1>

The monolithic ion exchanger No. 1 has a continuous macroporous structure having common openings (mesopores) with an average diameter within a range from 1 to 1,000 µm formed in the walls of mutually joined macropores, wherein the total pore volume is within a range from 1 to 50 mL/g, the ion exchange capacity per unit weight in a dry state is within a range from 1 to 9 mg equivalent/g, and the ion exchange groups are distributed throughout the organic porous ion exchanger.

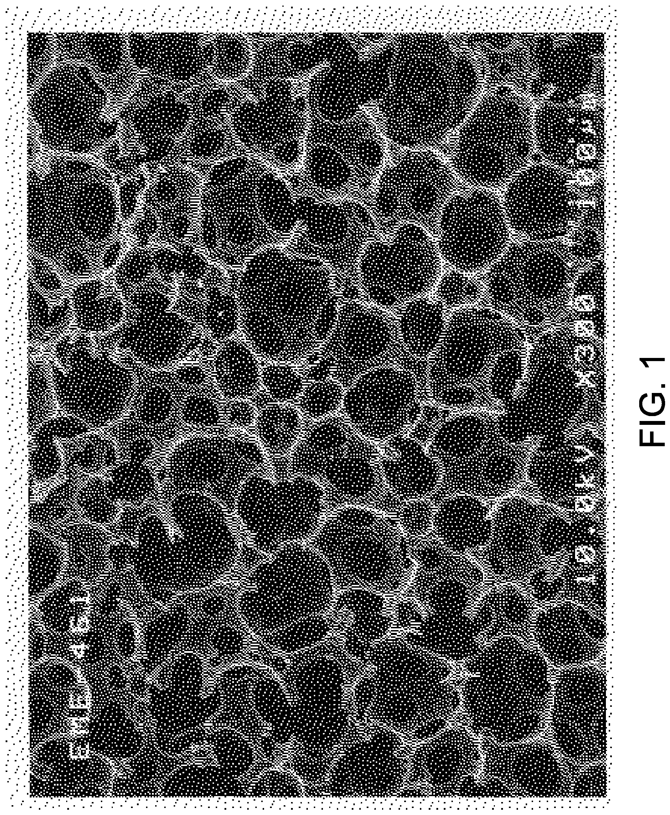

As illustrated in FIG. 1, the monolithic ion exchanger No. 1 is a continuous macroporous structure having continuous macropores (pores). The monolithic ion exchanger No. 1 and a production method therefor are disclosed in JP 2002-306976 A.

The monolithic ion exchanger No. 1 has common openings (mesopores) positioned in the walls between mutually joined macropores. These mesopores have overlapping portions where the macropores overlap one another. These overlapping portions of the mesopores have an average diameter in a dry state that is preferably within a range from 1 to 1,000 µm, more preferably within a range from 10 to 200 µm, and even more preferably from 20 to 200 µm. The average diameter of the openings of the monolith No. 1 in a dry state can be measured by mercury porosimetry, and indicates the value at the maximum of the pore distribution curve obtained by mercury porosimetry.

The majority of this type of monolithic ion exchanger No. 1 has an open-pore structure in which the spaces formed by the macropores and mesopores function as flow passages. If the average diameter of the overlapping portions of the mesopores in a dry state is less than 1 µm, then when the ion exchanger is packed in a column and a treatment target liquid is passed through the column, the pressure loss during liquid passage may sometimes increase markedly. If the average diameter of the overlapping portions of the mesopores in a dry state exceeds 1,000 µm, then when the ion exchanger is packed in a column and a treatment target liquid is passed through the column, the contact between the treatment target liquid and the monolithic ion exchanger may sometimes be unsatisfactory, causing a deterioration in the ion exchange performance. The number of overlaps between macropores may, for example, be within a range from 1 to 12 overlaps per macropore, with 3 to 10 overlaps for most macropores. Because the monolithic ion exchanger No. 1 is the type of continuous macroporous structure described above, the group of macropores and the group of common pores can be formed essentially uniformly, and compared with the type of particle aggregate-type porous body disclosed in JP H08-252579 A, the pore volume and the specific surface area can be increased dramatically.

The total pore volume per unit weight of the monolithic ion exchanger No. 1 in a dry state is preferably within a range from 1 to 50 mL/g, and more preferably within a range from 2 to 30 mL/g. If the total pore volume per unit weight in a dry state is less than 1 mL/g, then when the ion exchanger is packed in a column and a treatment target liquid is passed through the column, the pressure loss during liquid passage may sometimes increase, and moreover, the transmission amount per unit cross-sectional area may decrease, causing a fall in the processing capacity. If the total pore volume per unit weight in a dry state exceeds 50 mL/g, then the mechanical strength decreases, and when the ion exchanger is packed in a column and a treatment target liquid is passed through the column, the monolithic ion exchanger may sometimes deform, particularly when the liquid is passed through at a high flow rate.

The ion exchange capacity per unit weight in a dry state is as described above. Further, the fact that "the ion exchange groups are distributed throughout the organic porous ion exchanger" is also as described above.

### <Method for Producing Monolithic Ion Exchanger No. 1>

The monolithic ion exchanger No. 1 can be produced, for example, using the method described below.

For example, first, an oil-soluble monomer containing no ion exchange groups, a surfactant, water, and if necessary a polymerization initiator can be mixed together to obtain a water-in-oil emulsion. Next, this water-in-oil emulsion can be polymerized to form the monolith No. 1.

The oil-soluble monomer containing no ion exchange groups used in the production of the monolith No. 1 refers to an lipophilic monomer containing no ion exchange groups that exhibits low solubility in water. Examples of this monomer include styrene, α-methylstyrene, vinylbenzyl chloride, ethylene, propylene, vinyl chloride, vinyl bromide, acrylonitrile, methacrylonitrile, vinyl acetate, methyl acrylate, ethyl acrylate, 2-ethylhexyl acrylate, butanediol diacrylate, methyl methacrylate, ethyl methacrylate, 2-ethylhexyl methacrylate, and ethylene glycol dimethacrylate. One of these monomers may be used alone, or a combination of two or more monomers may be used. However, a crosslinking monomer such as divinylbenzene or ethylene glycol dimethacrylate is selected as at least one component of the oil-soluble monomer, and ensuring that the amount of that crosslinking monomer within the total oil-soluble monomer is, for example, within a range from 0.3 to 10 mol%, and preferably within a range from 0.3 to 5 mol%, is preferable in terms of ensuring quantitative introduction of the ion exchange groups in a later step, and ensuring a level of mechanical strength sufficient for practical purposes.

There are no particular limitations on the surfactant used in the production of the monolith No. 1, provided it is capable of forming a water-in-oil (W/O) emulsion when mixed with the oil-soluble monomer containing no ion exchange groups and water. Examples of the surfactant include nonionic surfactants such as sorbitan monooleate, sorbitan monolaurate and polyoxyethylene nonylphenyl ether; anionic surfactants such as potassium oleate, sodium dodecylbenzenesulfonate and dioctyl sodium sulfosuccinate; cationic surfactants such as distearyldimethylammonium chloride; and amphoteric surfactants such as lauryldimethylbetaine. One of these surfactants may be used alone, or a combination of two or more surfactants may be used. A water-in-oil emulsion refers to an emulsion in which the oil phase is the continuous phase, and water droplets are dispersed through that continuous oil phase. The amount added of the surfactant may be set, for example, to a value within a range from approximately 2 to 70% relative to the combined mass of the oil-soluble monomer and the surfactant. In order to control the shape and size of the bubbles in the monolith, an alcohol such as methanol or stearyl alcohol, a carboxylic acid such as stearic acid, a hydrocarbon such as octane, dodecane or toluene, or a cyclic ether such as tetrahydrofuran or dioxane may also be added to the production system.

In the production of the monolith No. 1, the polymerization initiator that may be used as necessary during the formation of the monolith by polymerization is ideally a compound that generates radicals upon heat or light irradiation. The polymerization initiator may be water-soluble or oil-soluble, and examples include azobisisobutyronitrile, azobisdimethylvaleronitrile, azobiscyclohexanenitrile, azobiscyclohexanecarbonitrile, benzoyl peroxide, potassium persulfate, ammonium persulfate, hydrogen peroxide-ferrous chloride, sodium persulfate-sodium hydrogen sulfite, and tetramethylthiuram disulfide. However, in some cases the polymerization may proceed without adding a polymerization initiator, simply by conducting heating or light irradiation, and in such systems a polymerization initiator need not be added.

In the production of the monolith No. 1, the polymerization conditions under which the water-in-oil emulsion is polymerized may be selected in accordance with a variety of factors such as type of monomer(s) and the initiator system and the like. When azobisisobutyronitrile, benzoyl peroxide, or potassium persulfate or the like is used as the polymerization initiator, a heated polymerization may be conducted, for example, in a sealed container under an inert atmosphere, for example, at a temperature of 30 to 100°C for a period of 1 to 48 hours. When hydrogen peroxide-ferrous chloride or sodium persulfate-sodium hydrogen sulfite or the like is used as the polymerization initiator, the polymerization may be conducted, for example, in a sealed container under an inert atmosphere, for example, at a temperature of 0 to 30°C for a period of 1 to 48 hours. Following completion of the polymerization, the contents may be removed and subjected to a Soxhlet extraction using a solvent such as isopropanol to remove any unreacted monomer and residual surfactant and obtain the monolith No. 1.

Examples of the method used for introducing ion exchange groups into the monolith No. 1 include the following methods (1) and (2). (1) By replacing the monomer containing no ion exchange groups, and instead conducting the polymerization using a monomer containing an ion exchange group, for example, a monomer prepared by introducing an ion exchange group into an aforementioned oil-soluble monomer containing no ion exchange groups, a monolithic ion exchanger can be produced in a single stage. (2) The monolith No. 1 is first formed by conducting a polymerization using a monomer containing no ion exchange groups, and ion exchange groups can then be introduced.

Examples of methods that may be used for introducing ion exchange groups into the monolith No. 1 include conventional methods such as polymer reactions and graft polymerizations. Examples of methods for introducing strongly basic anion exchange groups (quaternary ammonium groups) include the following methods (1) to (3). In method (1), if the monolith is a styrene-divinylbenzene-based copolymer or the like, chloromethyl groups are introduced using chloromethyl methyl ether or the like, and these groups may then be reacted with a desired tertiary amine to introduce the quaternary ammonium groups. In method (2), the monolith is produced by copolymerization of chloromethylstyrene and divinylbenzene, and the monolith may then be reacted with a desired tertiary amine to introduce the quaternary ammonium groups. In method (3), radical initiator groups or chain transfer groups are introduced into the monolith, glycidyl methacrylate is then graft polymerized, and a reaction may then be conducted with a desired tertiary amine to introduce the quaternary ammonium groups.

For example, a monolithic ion exchanger No. 1 can be produced by reacting a polymer chain represented by general formula (2):

[Formula 5] Polymer-L-X

(wherein L represents a linker site, "Polymer" represents a polymer chain, and X represents a halogen atom, an alkylsulfonyl group of 1 to 8 carbon atoms which may be substituted, or a benzenesulfonyl group which may be substituted), and a tertiary amine represented by general formula (3): 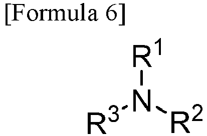 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; and R² and R³ each independently represent an alkyl group of 1 to 4 carbon atoms).

In this method for producing an ion exchanger, the definitions for L and Polymer in general formula (2), and the definitions for R¹, R² and R³ in general formula (3) are the same as the definitions for L, Polymer, R¹, R² and R³ respectively in general formula (1).

X in general formula (2) represents a halogen atom, an alkylsulfonyl group of 1 to 8 carbon atoms which may be substituted, or a benzenesulfonyl group which may be substituted.

Examples of the halogen atom for X include a chlorine atom, a bromine atom and an iodine atom, and in terms of factors such as reducing the expense of the production raw materials, a chlorine atom is preferred.

The alkyl group in the alkylsulfonyl group of 1 to 8 carbon atoms may be linear, branched or cyclic. In terms of factors such as the ease of availability of the raw material and achieving a high reaction yield during production, a methylsulfonyl group or an ethylsulfonyl group is preferred.

The alkylsulfonyl group of 1 to 8 carbon atoms may be substituted with a cyano group, a nitro group, or a halogen atom or the like, and in terms of achieving a high reaction yield and the like, a halogen atom is preferred, and a fluorine atom is more preferred.

The benzenesulfonyl group may be substituted with an alkyl group of 1 to 4 carbon atoms, a nitro group, a cyano group, or a halogen atom or the like, and in terms of factors such as the ease of availability of the raw material, a methyl group is preferred.

The reaction between the polymer chain represented by general formula (2) and the tertiary amine represented by general formula (3) is conducted, for example, by mixing the reactants in an organic solvent such as tetrahydrofuran, DMF or toluene, and then conducting heating, for example, at a temperature of 20 to 130°C for a period of 4 to 60 minutes. Following reaction, the product may be washed with an organic solvent such as methanol or tetrahydrofuran, or with water or the like.

### <Monolithic Ion Exchanger No. 2>

The monolithic ion exchanger No. 2 is formed as a three-dimensionally continuous skeleton portion composed of organic polymer particles with an average particle diameter within a range from 1 to 50 µm aggregated together, and has three-dimensionally continuous pores with an average diameter within a range from 20 to 100 µm within that skeleton, wherein the total pore volume is within a range from 1 to 10 mL/g, the ion exchange capacity per unit weight in a dry state is within a range from 1 to 9 mg equivalent/g, and the ion exchange groups are distributed throughout the organic porous ion exchanger.

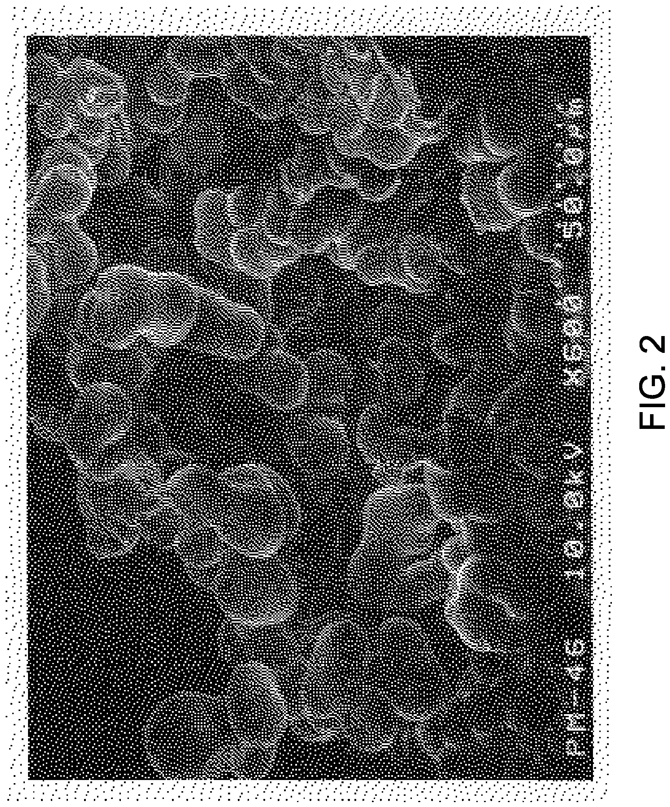

As illustrated in FIG. 2, the monolithic ion exchanger No. 2 is a particle aggregate-type structure composed of aggregated particles. The monolithic ion exchanger No. 2 and a production method therefor are disclosed in JP 2009-007550 A.

The monolithic ion exchanger No. 2 has a three-dimensionally continuous skeleton portion composed of aggregated organic polymer particles having crosslinked structural units and having an average particle diameter in a dry state that is preferably within a range from 1 to 50 µm, and more preferably within a range from 1 to 30 µm. The monolithic ion exchanger No. 2 has three-dimensionally continuous pores within that skeleton, with the pores having an average diameter in a dry state that is preferably within a range from 20 to 100 µm, and more preferably within a range from 20 to 90 µm. A SEM photograph is acquired of a randomly extracted portion of a cross-section of the monolithic ion exchanger No. 2 in a dry state, the diameters of all of the organic polymer particles within that SEM photograph are measured, and the average of those diameters is deemed the average particle diameter. The average diameter of the continuous pores in a dry state is determined by mercury porosimetry, in the same manner as described for the monolithic ion exchanger No. 1.

If the average particle diameter of the organic polymer particles in a dry state is less than 1 µm, then the average diameter in a dry state of the continuous pores within the skeleton may sometimes decrease to less than 20 µm. If the average particle diameter of the organic polymer particles exceeds 50 µm, then when the ion exchanger is packed into a column and a treatment target liquid is passed through the column, the pressure loss may sometimes increase. Further, if the average diameter of the continuous pores in a dry state is less than 20 µm, then when the ion exchanger is packed into a column and a treatment target liquid is passed through the column, the pressure loss during passage of the treatment target liquid may sometimes increase. If the average diameter of the continuous pores in a dry state exceeds 100 µm, then when the ion exchanger is packed into a column and a treatment target liquid is passed through the column, the contact between the treatment target liquid and the monolithic ion exchanger may sometimes become unsatisfactory.

The total pore volume per unit weight of the monolithic ion exchanger No. 2 in a dry state is preferably within a range from 1 to 10 mL/g. If the total pore volume is less than 1 mL/g, then when the ion exchanger is packed into a column and a treatment target liquid is passed through the column, the pressure loss during liquid passage may sometimes increase. Moreover, the transmission amount per unit cross-sectional area may decrease, causing a fall in the processing capacity. If the total pore volume exceeds 10 mL/g, then the mechanical strength decreases, and when the ion exchanger is packed in a column and a treatment target liquid is passed through the column, the monolithic ion exchanger may sometimes deform, particularly when the liquid is passed through at a high flow rate.

The ion exchange capacity per unit weight in a dry state is as described above. Further, the fact that "the ion exchange groups are distributed throughout the organic porous ion exchanger" is also as described above.

### <Method for Producing Monolithic Ion Exchanger No. 2>

The monolithic ion exchanger No. 2 can be produced, for example, using the method described below.

For example, the monolithic ion exchanger No. 2 can be obtained by mixing a vinyl monomer, a prescribed amount of a crosslinking agent, an organic solvent and a polymerization initiator, and then polymerizing this mixture in a stationary state.

The vinyl monomer used in the production of the monolith No. 2 is the same as the monomer used in the production of the monolith No. 1.

The crosslinking agent used in the production of the monolith No. 2 is preferably a compound having at least two polymerizable vinyl groups in each molecule and having good solubility in the organic solvent. Examples of the crosslinking agent include divinylbenzene, divinylbiphenyl and ethylene glycol dimethacrylate. One of these crosslinking agent may be used alone, or a combination of two or more crosslinking agents may be used. In terms of factors such as high mechanical strength and favorable stability relative to hydrolysis, preferred crosslinking agents include aromatic polyvinyl compounds such as divinylbenzene, divinylnaphthalene and divinylbiphenyl. The amount used of the crosslinking agent relative to the combined amount of the vinyl monomer and the crosslinking agent ({ crosslinking agent / (vinyl monomer + crosslinking agent)} × 100) is, for example, within a range from 1 to 5 mol%, and is preferably within a range from 1 to 4 mol%.

The organic solvent used in the production of the monolith No. 2 is a solvent that dissolves the vinyl monomer and the crosslinking agent, but in which the polymer produced by polymerization of the vinyl monomer is almost insoluble, or in other words, a poor solvent for the polymer produced by polymerization of the vinyl monomer. For example, in the case where the vinyl monomer is styrene, examples of this organic solvent include alcohols such as methanol, butanol and octanol, chain-like ethers such as diethyl ether and ethylene glycol dimethyl ether, and chain-like saturated hydrocarbons such as hexane, octane and decane.

The polymerization initiator used in the production of the monolith No. 2 is preferably a compound that generates radicals upon heating or light irradiation. The polymerization initiator is preferably oil-soluble. Examples of the polymerization initiator include 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobisisobutyrate, 4,4'-azobis(4-cyanovaleric acid), 1,1'-azobis(cyclohexane-1-carbonitrile), benzoyl peroxide, lauroyl peroxide, potassium persulfate, ammonium persulfate, and tetramethylthiuram disulfide. The amount used of the polymerization initiator relative to the combined amount of the vinyl monomer and the crosslinking agent ({polymerization initiator / (vinyl monomer + crosslinking agent)} × 100) is, for example, within a range from about 0.01 to 5 mol%.

In the production of the monolith No. 2, in those cases where, for example, 2,2'-azobis(isobutyronitrile), 2,2'-azobis(2,4-dimethylvaleronitrile), benzoyl peroxide, lauroyl peroxide, or potassium persulfate or the like is used as the polymerization initiator, a thermal polymerization may be conducted, for example, in a sealed container under an inert atmosphere, for example, at a temperature of 30 to 100°C for a period of 1 to 48 hours. Following completion of the polymerization, the contents may be removed and extracted with a solvent such as acetone to remove any unreacted vinyl monomer and the organic solvent, thus obtaining the monolith No. 2.

In the production of the monolith No. 2, by adjusting the polymerization conditions, for example, by increasing the amount of the crosslinking agent, increasing the monomer concentration, or increasing the temperature or the like, organic polymer particles having an average particle diameter of 1 to 50 µm can be aggregated. By using the prescribed amount of the crosslinking agent relative to the combined amount of the vinyl monomer and the crosslinking agent, three-dimensionally continuous pores having an average particle diameter of 20 to 100 µm can be formed within that skeleton. By conducting the polymerization under conditions in which, by and large, the amount used of the organic solvent relative to the combined amount of the organic solvent, the monomer and the crosslinking agent ({ organic solvent / (organic solvent + monomer + crosslinking agent)} × 100) is, for example, within a range from 30 to 80% by weight, and preferably within a range from 40 to 70% by weight, a total pore volume of the monolith within a range from 1 to 5 mL/g can be achieved.

The method used for introducing ion exchange groups into the monolith No. 2 may be the same as the methods used for introducing ion exchange groups into the monolith No. 1.

### <Monolithic Ion Exchanger No. 3>

The monolithic ion exchanger No. 3 is a continuous macroporous structure in which bubble-shaped macropores overlap with each other, with these overlapping portions forming openings having an average diameter within a range from 30 to 300 µm, wherein the total pore volume is within a range from 0.5 to 10 mL/g, the ion exchange capacity per unit weight in a dry state is within a range from 1 to 9 mg equivalent/g, the ion exchange groups are distributed throughout the organic porous ion exchanger, and the surface area of the skeleton portion shown in cross-section, in an SEM image of a cross-section of the continuous macroporous structure, is within a range from 25 to 50% within the image region.

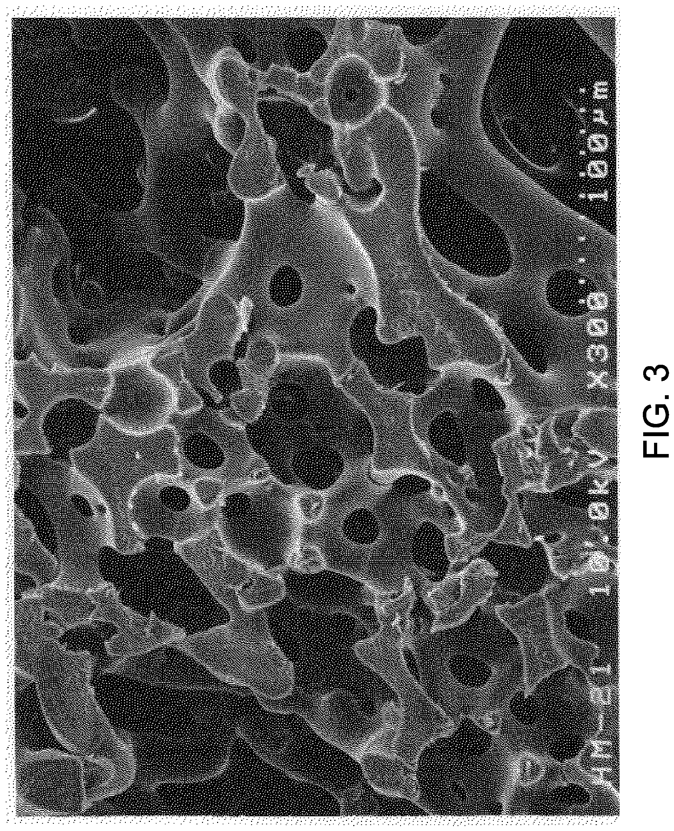

As illustrated in FIG. 3, the monolithic ion exchanger No. 3 is a continuous macroporous structure similar to the monolithic ion exchanger No. 1. The monolithic ion exchanger No. 3 and a production method therefor are disclosed in JP 2009-062512 A.

The continuous pores have overlapping portions where macropores overlap with each other. These overlapping portions have an average diameter in a dry state that is preferably within a range from 30 to 300 µm, more preferably within a range from 30 to 200 µm, and even more preferably within a range from 40 to 100 µm. This average diameter can be measured by mercury porosimetry, and indicates the value at the maximum of the pore distribution curve obtained by mercury porosimetry. If the average diameter of the openings in a dry state is less than 30 µm, then when the ion exchanger is packed into a column and a treatment target liquid is passed through the column, the pressure loss during liquid passage may sometimes increase, whereas if the average diameter exceeds 300 µm, then the contact between the treatment target liquid and the monolithic ion exchanger may sometimes become unsatisfactory.

In the monolithic ion exchanger No. 3, in a SEM image of a cross-section of the continuous macroporous structure in a dry state, the surface area of the skeleton portion shown in cross-section represents, for example, a percentage within a range from 20 to 50%, and preferably within a range from 25 to 45%, of the entire image region. If the surface area of the skeleton portion shown in cross-section is less than 25% of the entire image region, then the skeleton is thin and the mechanical strength decreases, and when the ion exchanger is packed into a column and a treatment target liquid is passed through the column, the monolithic ion exchanger may sometimes deform, particularly when the liquid is passed through at a high flow rate. If the surface area of the skeleton portion shown in cross-section exceeds 50% of the entire image region, then the skeleton becomes too thick, and when the ion exchanger is packed into a column and a treatment target liquid is passed through the column, the pressure loss during liquid passage may sometimes increase.

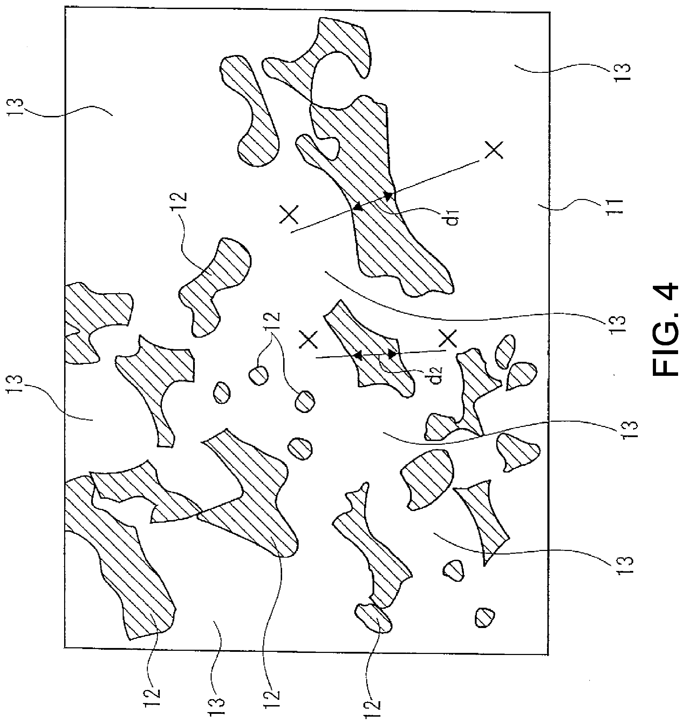

The conditions used for obtaining the SEM image may be any conditions that enable the skeleton portion to be shown clearly in the cross-section of a cut section, and examples of the conditions include a magnification of 100 to 600×, and a photograph region of approximately 150 mm × 100 mm. The SEM observation may be conducted using three or more images acquired at non-subjective arbitrary locations of an arbitrary cross-section of the monolithic ion exchanger No. 3. The cut monolithic ion exchanger No. 3 is in a dry state. The skeleton portion of the cross-section in the SEM image is described below using FIG. 3 and FIG. 4. In FIG. 3 and FIG. 4, the portion of irregular form that is also shown in the cross-section is the "skeleton portion shown in cross-section" (reference sign 12), the circular holes shown in FIG. 3 are openings (mesopores), and the objects having comparatively large curvature and curved surfaces are macropores (reference sign 13 in FIG. 4). The surface area of the skeleton portion shown in cross-section in FIG. 4 is 28% of the rectangular image region 11.

In the SEM image, examples of methods that may be used for measuring the surface area of the skeleton portion shown in cross-section within the cut section include calculation methods in which the skeleton portion is defined by performing conventional computer processing or the like, and the surface area is then calculated by either an automated calculation using a computer or the like or by manual calculation. In one example of a manual calculation method, the irregular forms are converted to combinations of squares, triangles, circles and trapezoids and the like, and the areas of these objects are combined to determine the surface area.

The total pore volume per unit weight of the monolithic ion exchanger No. 3 in a dry state is preferably within a range from 0.5 to 10 mL/g, and more preferably within a range from 0.8 to 8 mL/g. If the total pore volume is less than 0.5 mL/g, then when the ion exchanger is packed in a column and a treatment target liquid is passed through the column, the pressure loss during liquid passage may sometimes increase, and moreover, the transmitted fluid volume per unit cross-sectional area may decrease, causing a fall in the processing capacity. If the total pore volume exceeds 10 mL/g, then the mechanical strength decreases, and when the ion exchanger is packed in a column and a treatment target liquid is passed through the column, the monolithic ion exchanger may sometimes deform, particularly when the liquid is passed through at a high flow rate. Moreover, the contact efficiency between the treatment target liquid and the monolithic ion exchanger may sometimes deteriorate.

The ion exchange capacity per unit weight in a dry state is as described above. Further, the fact that "the ion exchange groups are distributed throughout the organic porous ion exchanger" is also as described above.

### <Method for Producing Monolithic Ion Exchanger No. 3>

The monolithic ion exchanger No. 3 can be produced, for example, using the method described below.

For example, first, a mixture of an oil-soluble monomer containing no ion exchange groups, a surfactant and water is stirred to prepare a water-in-oil emulsion. Next, by conducting a step I, step II and step III described below, the monolith No. 3 can be obtained. In step I, the water-in-oil emulsion is polymerized to obtain, for example, a monolithic organic porous intermediate (hereinafter also referred to as the monolith intermediate (3)) with a continuous macroporous structure having a total pore volume within a range from 5 to 16 mL/g. In step II, a mixture is prepared containing a vinyl monomer, a crosslinking agent having at least two vinyl groups within each molecule, an organic solvent that dissolves the vinyl monomer and the crosslinking agent but does not dissolve the polymer produced by polymerization of the vinyl monomer, and a polymerization initiator. In step III, the mixture obtained in step II is subjected to polymerization in a stationary state and in the presence of the monolith intermediate (3) obtained in step I, thus obtaining a monolith No. 3 having a thicker skeleton than the skeleton of the monolith intermediate (3).

Step I is the same as step I in the method for producing the monolithic ion exchanger No. 1.

The monolith intermediate (3) obtained in step I has a continuous macroporous structure. By including this intermediate in the polymerization system, a porous structure having a thick-walled skeleton can be formed using the structure of the monolith intermediate (3) as a matrix. The crosslinking density of the polymer material is such that, relative to all of the structural units that constitute the polymer material of the monolith intermediate (3), the number of crosslinked structural units is, for example, within a range from 0.3 to 10 mol%, and preferably within a range from 0.3 to 5 mol%.

The total pore volume per unit weight in a dry state of the monolith intermediate (3) obtained in step I is, for example, within a range from 5 to 16 mL/g, and preferably within a range from 6 to 16 mL/g. In order to ensure that the total pore volume of the monolith intermediate (3) falls within the above numerical range, the ratio between the monomer and water is, for example, within a range from about 1:5 to 1:20.

In the monolith intermediate (3) obtained in step I, the average diameter in a dry state of the openings (mesopores) that represent the overlapping portions between macropores is, for example, within a range from 20 to 200 µm.

Step II is a step of preparing a mixture containing a vinyl monomer, a crosslinking agent having at least two vinyl groups within each molecule, an organic solvent that dissolves the vinyl monomer and the crosslinking agent but does not dissolve the polymer produced by polymerization of the vinyl monomer, and a polymerization initiator. Either step I or step II may be conducted first.

The vinyl monomer used in step II may be any lipophilic vinyl monomer containing a polymerizable vinyl group within the molecule and having good solubility in the organic solvent, and selection of a vinyl monomer that produces a polymer material of the same type or a similar type to the monolith intermediate (3) included in the polymerization system is preferred. Specific examples of these vinyl monomers are the same as those listed above for the vinyl monomer used in the production of the monolith No. 1.

The amount added of the vinyl monomer used in step II, expressed as a weight, is typically within a range from 3 to 50 times, and preferably within a range from 4 to 40 times, the weight of the monolith intermediate (3) that is also included during the polymerization.

The crosslinking agent used in step II is the same as the crosslinking agent used in the production of the monolith No. 2.

The organic solvent used in step II is the same as the organic solvent used in the production of the monolith No. 2. The amount used of these organic solvents is preferably determined so that the concentration of the above vinyl monomer is, for example, within a range from 30 to 80% by weight.

The polymerization initiator used in step II is the same as the polymerization initiator used in the production of the monolith No. 2.

In step III, for example, the mixture obtained in step II is subjected to stationary polymerization in the presence of the monolith intermediate (3) obtained in step I, enabling a monolith No. 3 having a thicker skeleton than the skeleton of the monolith intermediate (3) to be obtained. By including the monolith intermediate (3) with a continuous macroporous structure in the above polymerization system, the monolith No. 3 can be obtained.

In step III, for example, the monolith intermediate (3) is placed in a reaction container in a state impregnated with the mixture (solution). The blend ratio between the mixture obtained in step II and the monolith intermediate (3) is adjusted so that, for example, the amount added of the vinyl monomer, expressed as a weight, is within a range from 3 to 50 times, and preferably within a range from 4 to 40 times, the weight of the monolith intermediate (3). In the reaction container, the vinyl monomer and the crosslinking agent in the mixture adsorb to and are distributed across the skeleton of the stationary monolith intermediate, with the polymerization then progressing within the skeleton of the monolith intermediate (3).

In step III, various polymerization conditions may be selected depending on the type of monomer used and the type of polymerization initiator and the like. For example, in those cases where 2,2'-azobis(isobutyronitrile), 2,2'-azobis(2,4-dimethylvaleronitrile), benzoyl peroxide, lauroyl peroxide, or potassium persulfate or the like is used as the polymerization initiator, a thermal polymerization may be conducted, for example, in a sealed container under an inert atmosphere, for example, at a temperature of 30 to 100°C for a period of 1 to 48 hours. As a result of this thermal polymerization, the vinyl monomer and crosslinking agent that have adsorbed to and are distributed across the skeleton of the monolith intermediate (3) undergo polymerization within the skeleton, enabling a thickening of the skeleton. Following completion of the polymerization, the contents may be removed and extracted with a solvent such as acetone to remove any unreacted vinyl monomer and the organic solvent, thus obtaining the monolith No. 3.

The monolithic ion exchanger No. 3 can be obtained, for example, by conducting a step IV of introducing ion exchange groups into the monolith No. 3 obtained in step III. The method used for introducing ion exchange groups into the monolith No. 3 may be the same as the methods used for introducing ion exchange groups into the monolith No. 1.

### <Monolithic Ion Exchanger No. 4>

The monolithic ion exchanger No. 4 is a co-continuous structure composed of a three-dimensionally continuous skeleton in which the continuous skeleton, formed from an aromatic vinyl polymer containing from 0.1 to 5.0 mol% of crosslinked structural units among all of the structural units having an introduced ion exchange group, has a thickness within a range from 1 to 60 µm, and three-dimensionally continuous pores with an average diameter within a range from 10 to 200 µm within that skeleton, wherein the total pore volume is within a range from 0.5 to 10 mL/g, the ion exchange capacity per unit weight in a dry state is within a range from 1 to 9 mg equivalent/g, the ion exchange groups are distributed throughout the organic porous ion exchanger.

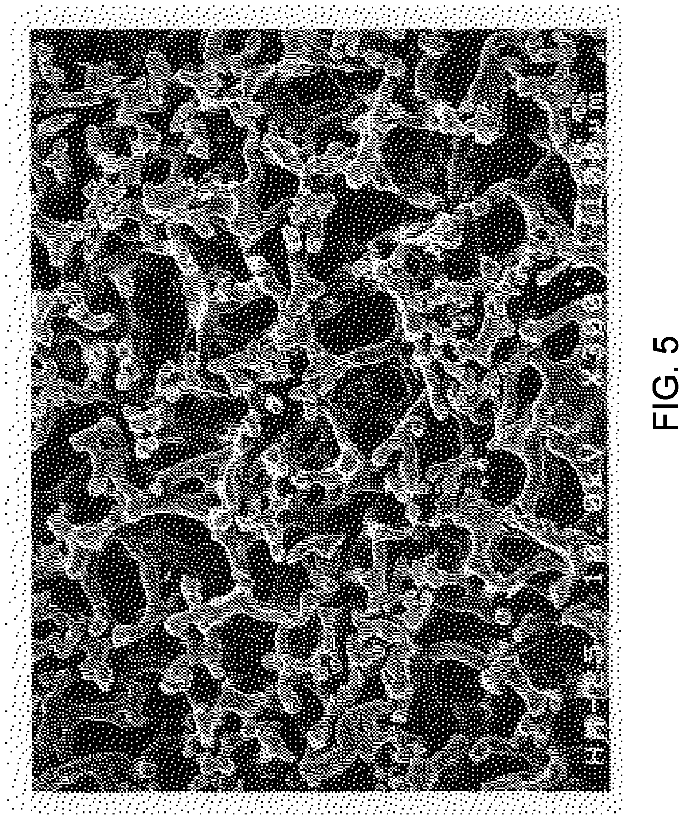

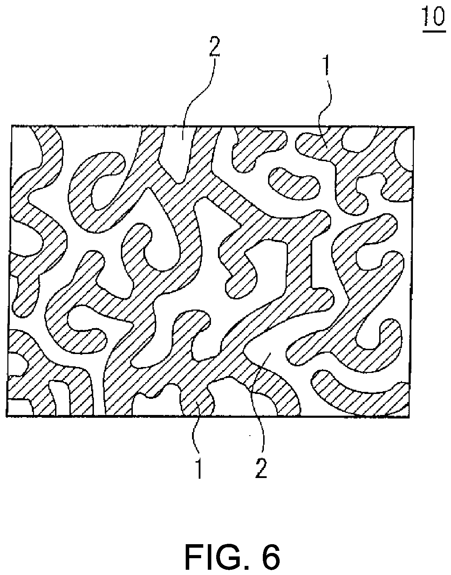

As illustrated in FIG. 5 and FIG. 6, the monolithic ion exchanger No. 4 has a continuous skeleton phase 1 (the continuous skeleton) and a continuous pore phase 2 (the continuous pores), and those phases intertwine and form a co-continuous structure 10 in which each phase is three-dimensionally continuous. The pore phase 2 has higher continuity than the monoliths No. 1 and No. 2 described above, and has almost no variation in size. It is though that the monolithic ion exchanger No. 4 has a high mechanical strength due to its thick skeleton. The monolithic ion exchanger No. 4 and a production method therefor are disclosed in JP 2009-067982 A.

The continuous skeleton is composed of a vinyl polymer (such as an aromatic vinyl polymer) that contains from 0.1 to 5.0 mol% of crosslinked structural units among all of the structural units having an introduced ion exchange group, and is a three-dimensionally continuous structure in which the thickness of the continuous skeleton in a dry state is, for example, within a range from 1 to 60 µm, and preferably within a range from 3 to 58 µm. If the amount of crosslinked structural units is less than 0.1 mol%, then the mechanical strength may sometimes be unsatisfactory, whereas if the amount exceeds 5.0 mol%, the structure of the porous body is more likely to diverge from the co-continuous structure. If the thickness of the continuous skeleton in a dry state is less than 1 µm, then when the ion exchanger is packed into a column and a treatment target liquid is passed through the column, the monolithic ion exchanger may sometimes deform, particularly when the liquid is passed through at a high flow rate. If the thickness of the continuous skeleton in a dry state exceeds 60 µm, then the skeleton may become too thick, and when the ion exchanger is packed into a column and a treatment target liquid is passed through the column, the pressure loss during liquid passage may sometimes increase.

The continuous pores are three-dimensionally continuous within the continuous skeleton, and have an average diameter in a dry state that is, for example, within a range from 10 to 200 µm, and preferably within a range from 15 to 180 µm. If the average diameter of these continuous pores in a dry state is less than 10 µm, then when the ion exchanger is packed into a column and a treatment target liquid is passed through the column, the pressure loss during liquid passage may sometimes increase. If the average diameter exceeds 200 µm, then when the ion exchanger is packed into a column and a treatment target liquid is passed through the column, the contact between the treatment target liquid and the monolithic ion exchanger may sometimes be unsatisfactory.

The average diameter mentioned above can be measured by mercury porosimetry, and indicates the value at the maximum of the pore distribution curve obtained by mercury porosimetry. The thickness of the continuous skeleton in a dry state can be determined by SEM observation of the monolithic ion exchanger No. 4 in a dry state. Specifically, SEM observation of the monolithic ion exchanger No. 4 in a dry state is conducted at least three times, the skeleton thickness is measured in each of the obtained images, and the average value of those measurement is deemed the thickness of the continuous skeleton. The skeleton is rod-shaped with a circular cross-sectional shape, but also includes different diameter sections such as oval cross-sectional shapes. In such cases, the thickness is deemed the average of the minor axis and the major axis.

The total pore volume per unit weight of the monolithic ion exchanger No. 4 in a dry state is, for example, within a range from 0.5 to 10 mL/g. If the total pore volume is less than 0.5 mL/g, then when the ion exchanger is packed into a column and a treatment target liquid is passed through the column, the pressure loss during liquid passage may sometimes increase. Moreover, the transmitted fluid volume per unit cross-sectional area may decrease, causing a fall in the processing capacity. If the total pore volume exceeds 10 mL/g, then the mechanical strength decreases, and when the ion exchanger is packed in a column and a treatment target liquid is passed through the column, the monolithic ion exchanger may sometimes deform, particularly when the liquid is passed through at a high flow rate. Moreover, the contact efficiency between the treatment target liquid and the monolithic ion exchanger may sometimes deteriorate.

Examples of the vinyl polymer (aromatic vinyl polymer) that constitutes the continuous skeleton include polystyrene, poly(α-methylstyrene) and polyvinylbenzyl chloride. This polymer may be a polymer obtained by copolymerizing a single vinyl monomer and a crosslinking agent, a polymer obtained by copolymerizing a plurality of vinyl monomers and a crosslinking agent, or a blend of two or more polymers. Among these organic polymer materials, in terms of the ease of formation of the co-continuous structure, the ease of introduction of ion exchange groups, the mechanical strength, and the stability relative to acid or alkali, a styrene-divinylbenzene-based copolymer or a vinylbenzyl chloride-divinylbenzene-based copolymer is preferred.

The ion exchange capacity per unit weight in a dry state is as described above. Further, the fact that "the ion exchange groups are distributed throughout the organic porous ion exchanger" is also as described above.

### <Method for Producing Monolithic Ion Exchanger No. 4>

The monolithic ion exchanger No. 4 can be produced, for example, using the method described below.

For example, the monolith No. 4 can be obtained by preparing a water-in-oil emulsion, and then conducting steps I to III described below. In step I, for example, the water-in-oil emulsion is polymerized to obtain a monolithic organic porous intermediate (hereinafter also referred to as the monolith intermediate (4)) with a continuous macroporous structure having a total pore volume which, for example, exceeds 16 mL/g but is not more than 30 mL/g. In step II, a mixture is prepared containing, for example, an aromatic vinyl monomer, a crosslinking agent having at least two vinyl groups within each molecule, which is included in an amount, for example, within a range from 0.3 to 5 mol% of all of the oil-soluble monomers, an organic solvent that dissolves the aromatic vinyl monomer and the crosslinking agent but does not dissolve the polymer produced by polymerization of the aromatic vinyl monomer, and a polymerization initiator. In step III, for example, the mixture obtained in step II is subjected to polymerization in a stationary state and in the presence of the monolith intermediate (4) obtained in step I, thus obtaining a monolith No. 4.

Step I in this method for producing the monolith No. 4 is the same as step I in the method for producing the monolithic ion exchanger No. 1.

The monolith intermediate (4) obtained in step I is, for example, an organic polymer material having a crosslinked structure, and is preferably an aromatic vinyl polymer. The crosslinking density of this polymer material is such that, relative to all of the structural units that constitute the polymer material, the number of crosslinked structural units is, for example, within a range from 0.1 to 5 mol%, and preferably within a range from 0.3 to 3 mol%.

The types of polymer materials for the monolith intermediate (4) are the same as the types of polymer materials for the monolith intermediate (3) described above in the method for producing the monolith No. 3.

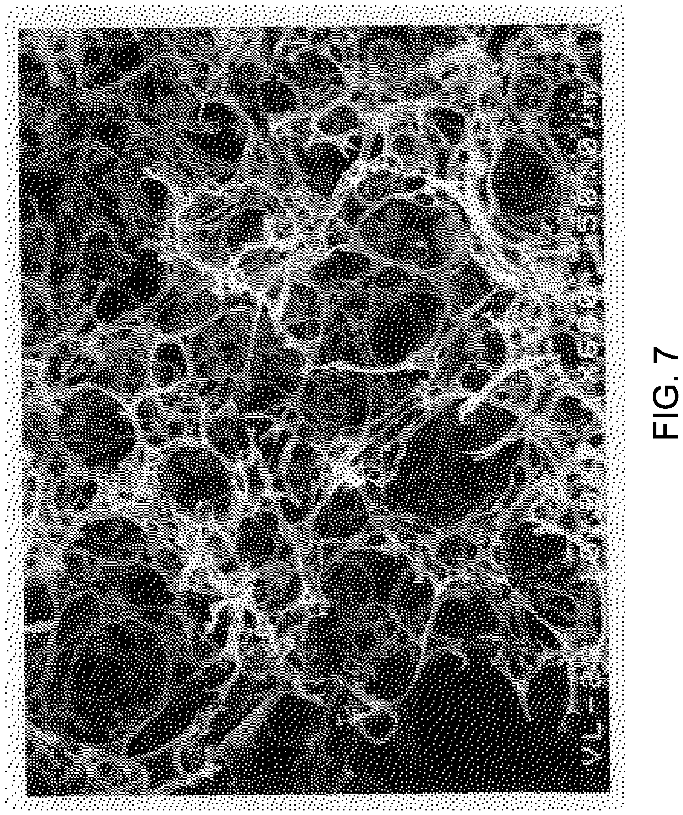

The total pore volume per unit weight in a dry state of the monolith intermediate (4) obtained in step I, for example, exceeds 16 mL/g but is not more than 30 mL/g, and preferably exceeds 16 mL/g but is not more than 25 mL/g. As illustrated in FIG. 7, the monolith intermediate (4) has a skeleton close to a rod-shaped structure. By including this intermediate in the polymerization system, a porous body having a co-continuous structure can be formed using the structure of the monolith intermediate (4) as a matrix.

In the monolith intermediate (4) obtained in step I, the average diameter in a dry state of the openings (mesopores) that represent the overlapping portions between macropores is, for example, within a range from 5 to 100 µm.

Step II in the method for producing the monolith No. 4 is a step of preparing a mixture containing, for example, an aromatic vinyl monomer, a crosslinking agent having at least two vinyl groups within each molecule, which is included in an amount, for example, within a range from 0.3 to 5 mol% of all of the oil-soluble monomers, an organic solvent that dissolves the aromatic vinyl monomer and the crosslinking agent but does not dissolve the polymer produced by polymerization of the aromatic vinyl monomer, and a polymerization initiator. Either step I or step II may be conducted first.

The aromatic vinyl monomer used in step II may be any lipophilic aromatic vinyl monomer containing a polymerizable vinyl group within the molecule and having good solubility in the organic solvent, and although there are no particular limitations, selection of a vinyl monomer that produces a polymer material of the same type or a similar type to the monolith intermediate (4) included in the polymerization system is preferred. Specific examples of the vinyl monomer include styrene, α-methylstyrene, vinyltoluene, vinylbenzyl chloride, vinylbiphenyl and vinylnaphthalene. One of these monomers may be used alone, or a combination of two or more monomers may be used. Examples of aromatic vinyl monomers that can be used particularly favorably include styrene and vinylbenzyl chloride.

The amount added of the aromatic vinyl monomer used in step II, expressed as a weight, is typically within a range from 5 to 50 times, and preferably within a range from 5 to 40 times, the weight of the monolith intermediate (4) that is also included during the polymerization.

The crosslinking agent used in step II is preferably a compound having at least two polymerizable vinyl groups in each molecule and having good solubility in the organic solvent. Examples of the crosslinking agent include divinylbenzene, divinylnaphthalene, divinylbiphenyl, ethylene glycol dimethacrylate, trimethylolpropane triacrylate, and butanediol diacrylate. One of these crosslinking agent may be used alone, or a combination of two or more crosslinking agents may be used. The amount used of the crosslinking agent relative to the combined amount of the vinyl monomer and the crosslinking agent (all of the oil-soluble monomers) is, for example, within a range from 0.3 to 5 mol%, and is preferably within a range from 0.3 to 3 mol%. The amount used of the crosslinking agent is preferably approximately equal to the crosslinking density of the monolith intermediate (4) that is also included during the vinyl monomer/crosslinking agent polymerization. If the two amounts are very different, then variation is more likely to develop in the crosslinking density distribution in the produced monolith, and when the ion exchange groups are introduced, cracks may sometimes occur more readily during the reaction for introducing the ion exchange groups.

The organic solvent used in step II is, for example, an organic solvent that dissolves the aromatic vinyl monomer and the crosslinking agent but does not dissolve the polymer produced by polymerization of the aromatic vinyl monomer, or in other words, a poor solvent for the polymer produced by polymerization of the aromatic vinyl monomer. For example, in the case where the aromatic vinyl monomer is styrene, examples of this organic solvent include alcohols such as methanol, butanol and octanol, chain-like (poly)ethers such as diethyl ether, polyethylene glycol and polypropylene glycol, chain-like saturated hydrocarbons such as hexane, heptane and octane, and esters such as ethyl acetate, isopropyl acetate, and ethyl propionate. Further, even good solvents of polystyrene such as dioxane, THF and toluene may be used as the organic solvent, provided they are used in combination with an aforementioned poor solvent in a relatively small amount. The amount used of these organic solvents is determined so that the concentration of the above aromatic vinyl monomer is, for example, within a range from 30 to 80% by weight. If the amount used of the organic solvent falls outside this range and the aromatic vinyl monomer concentration is less than 30% by weight, then the polymerization rate can sometimes decrease, and the monolith structure following the polymerization may deviate from the specified ranges for the monolith No. 4. On the other hand, if the aromatic vinyl monomer concentration exceeds 80% by weight, then the polymerization may sometimes proceed too readily.

The polymerization initiator used in step II in the method for producing the monolith No. 4 is the same as the polymerization initiator used in step II in the method for producing the monolith No.3.

Step III in the method for producing the monolith No. 4 is, for example, a step of subjecting the mixture obtained in step II to stationary polymerization in the presence of the monolith intermediate (4) obtained in step I, thereby converting the continuous macroporous structure of the monolith intermediate (4) to a co-continuous structure and enabling a monolith No. 4 having a co-continuous structure to be obtained.

In step III, for example, the monolith intermediate (4) is placed in a reaction container in a state impregnated with the mixture (solution). The blend ratio between the mixture obtained in step II and the monolith intermediate (4) may be adjusted so that, as described above, the amount added of the aromatic vinyl monomer, expressed as a weight, is within a range from 5 to 50 times, and preferably within a range from 5 to 40 times, the weight of the monolith intermediate (4). As a result, a monolith No. 4 can be obtained which has a co-continuous structure in which pores of an appropriate size are three-dimensionally continuous, and a thick-walled skeleton also forms a three-dimensionally continuous structure. In the reaction container, the aromatic vinyl monomer and the crosslinking agent in the mixture adsorb to and are distributed across the skeleton of the stationary monolith intermediate (4), with the polymerization then progressing within the skeleton of the monolith intermediate (4).

The polymerization conditions in step III in the method for producing the monolith No. 4 are the same as those described above for the polymerization conditions in step III in the method for producing the monolith No. 3. The monolithic ion exchanger No. 4 can be obtained by conducting a step IV of introducing ion exchange groups into the monolith No. 4 obtained in step III. The method used for introducing ion exchange groups into the monolith No. 4 may be the same as the methods used for introducing ion exchange groups into the monolith No. 1.

### <Monolithic Ion Exchanger No. 5>

The monolithic ion exchanger No. 5 is composed of a continuous skeleton phase and a continuous pore phase, wherein the skeleton has a plurality of particle bodies with a diameter within a range from 4 to 40 µm adhered to the skeleton surface or a plurality of protrusions with a size within a range from 4 to 40 µm formed on the surface of the skeleton of the organic porous body, the average diameter of the continuous pores is within a range from 10 to 200 µm, the total pore volume is within a range from 0.5 to 10 mL/g, the ion exchange capacity per unit weight in a dry state is within a range from 1 to 9 mg equivalent/g, and the ion exchange groups are distributed throughout the organic porous ion exchanger.

The monolithic ion exchanger No. 5 is a composite structure having an organic porous body with a continuous skeleton phase and a continuous pore phase, as well as a plurality of particle bodies or a plurality of protrusions, and is a composite structure having a multitude of particle bodies or a multitude of protrusions. The monolithic ion exchanger No. 5 and a production method therefor are disclosed in JP 2009-108294 A.

The plurality of particle bodies are adhered to the skeleton surface of the organic porous body, and the diameter of those particle bodies is, for example, within a range from 4 to 40 µm. The plurality of protrusions are formed on the surface of the skeleton of the organic porous body, and the size of those protrusions in a dry state is, for example, within a range from 4 to 40 µm. The diameter of the particle bodies or the size of the protrusions is preferably within a range from 4 to 30 µm, and more preferably within a range from 4 to 20 µm. In this description, the "particle bodies" and "protrusions" may be jointly referred to as "particle bodies or the like".

The average diameter of the continuous pores in a dry state is preferably within a range from 10 to 200 µm.

The continuous skeleton phase and the continuous pore phase of the monolithic ion exchanger No. 5 can be observed in SEM images. Examples of the basic structure of the monolithic ion exchanger No. 5 include continuous macroporous structures and co-continuous structures. The skeleton phase of the monolithic ion exchanger No. 5 appears as a column-like continuous body, a continuous body of concave wall surfaces, or a composite body thereof, and therefore the particle bodies or protrusions are of a clearly different shape.

The monolithic ion exchanger No. 5 includes a monolithic ion exchanger No. 5-1 and a monolithic ion exchanger No. 5-2. The monolithic ion exchanger No. 5-1 is a continuous macroporous structure in which bubble-shaped macropores overlap with each other, with these overlapping portions forming openings having an average diameter in a dry state that is, for example, within a range from 10 to 120 µm. The monolithic ion exchanger No. 5-2 is a co-continuous structure composed of a three-dimensionally continuous skeleton in which the thickness of the continuous skeleton in a dry state is, for example, within a range from 0.8 to 40 µm, and three-dimensionally continuous pores within that skeleton, with the pores having an average diameter in a dry state that is, for example, within a range from 8 to 80 µm. The monoliths of the monolithic ion exchangers No. 5-1 and No. 5-2 prior to the introduction of the ion exchange groups are also called the monoliths No. 5-1 and No. 5-2. The average diameter and the thickness of the continuous skeleton in a dry state mentioned above may be determined using the same measurement methods as those described for the monolithic ion exchanger No. 4.

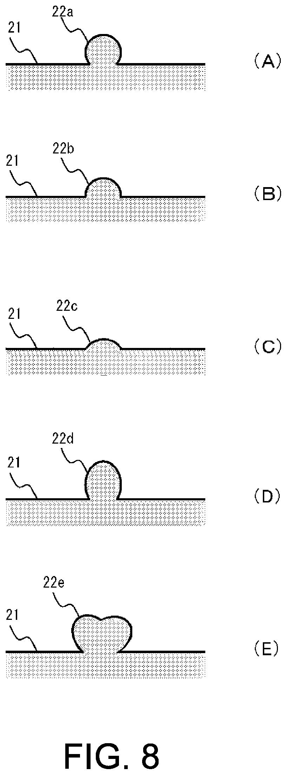

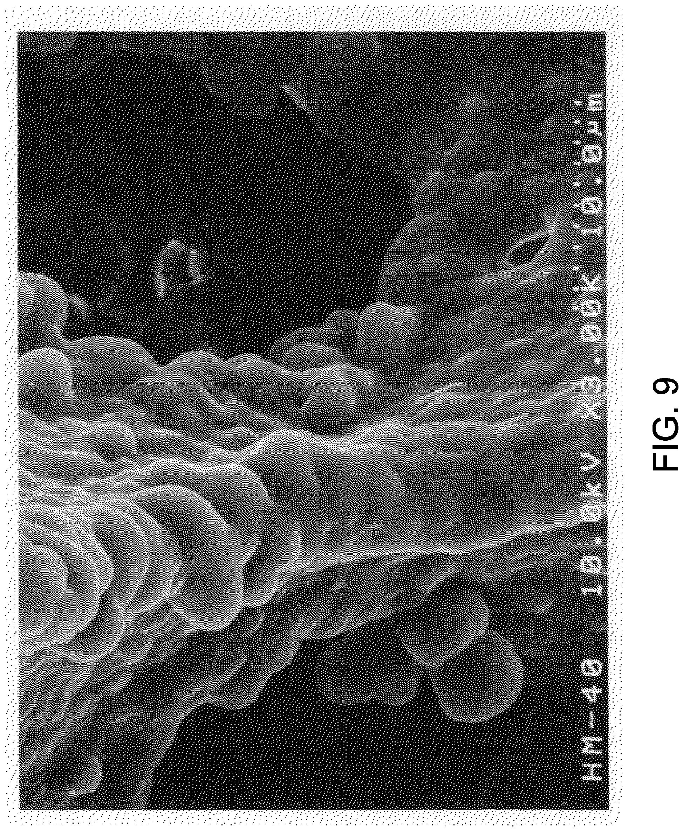

As illustrated in (A) to (E) of FIG. 8, the projection-like portions protruding from the skeleton surface 21 are protrusions 22a to 22e. As illustrated in (A), the protrusion 22a has a shape similar to that of a particle. As illustrated in (B), the protrusion 22b has a hemispherical shape. As illustrated in (C), the protrusion 22c has a shape similar to a bump in the skeleton surface. As illustrated in (D), the protrusion 22d has a shape with a length in the direction perpendicular to the skeleton surface 21 of the protrusion 22d that is longer than the length along the surface direction of the skeleton surface 21. As illustrated in (E), the protrusion 22e has a shape that protrudes in multiple directions. The size of each protrusion refers to the length across the portion where the protrusion width is greatest in a SEM image of the protrusion. As illustrated in FIG. 9, the monolithic ion exchanger 5 has a plurality of protrusions formed on the skeleton surface of the organic porous body.

In the monolithic ion exchanger No. 5, among all of the particle bodies or the like, the proportion of particle bodies or the like having a diameter in a dry state within the range from 4 to 40 µm is, for example, at least 70%, and preferably 80% or higher. The proportion of these particle bodies or the like indicates the numerical proportion of particles bodies or the like having a size in a dry state that satisfies the prescribed range among the total number of all the particle bodies or the like. Further, the particle bodies or the like typically cover, for example, at least 40%, and preferably 50% or more of the surface of the skeleton phase. The proportion of coverage of the surface of the skeleton layer by the particle bodies or the like indicates the surface area proportion in a SEM image when the surface is observed using a SEM, namely, the surface area proportion when the surface is viewed in plan view. If the size of the particles covering the wall surfaces and skeleton fall outside the above range, then the effect of the particles in improving the contact efficiency between the fluid and the skeleton surface and skeleton interior of the monolithic ion exchanger may sometimes diminish.

SEM observation of the monolithic ion exchanger No. 5 in a dry state is conducted at least three times, and for each field of view, the diameter or size in a dry state is calculated for all of the particle bodies or the like in each of the obtained images, a confirmation is made as to whether or not particle bodies or the like having a diameter or size that is, for example, within a range from 4 to 40 µm have been observed, and when this confirmation is made for all the fields of view, a determination is made that particle bodies or the like having a diameter or size in a dry state that is, for example, within a range from 4 to 40 µm are formed on the skeleton surface of the monolithic ion exchanger No. 5. Further, in accordance with the method described above, the diameter or size in a dry state of all of the particle bodies or the like in the SEM image of each field of view is calculated, the proportion of particle bodies or the like having a diameter or size in a dry state that is, for example, within a range from 4 to 40 µm among all of the particle bodies or the like is determined, and when this proportion of particle bodies or the like having a diameter or size in a dry state that is, for example, within a range from 4 to 40 µm among all of the particle bodies or the like is at least 70% in all the fields of view, a determination is made that among all of the particle bodies or the like formed on the skeleton surface of the monolithic ion exchanger No. 5, the proportion of particle bodies or the like having a diameter or size in a dry state that is, for example, within a range from 4 to 40 µm is at least 70%. Furthermore, in accordance with the method described above, the proportion of coverage of the surface of the skeleton layer by all of the particle bodies or the like is determined for the SEM image of each field of view, and when this proportion of coverage of the surface of the skeleton layer by all of the particle bodies or the like is at least 40% for all the fields of view, a determination is made that the proportion of the surface of the skeleton layer of the monolithic ion exchanger No. 5 covered by the particle bodies or the like is at least 40%.

In the monolithic ion exchanger No. 5, if the coverage rate of the skeleton phase surface by the particle bodies or the like is less than 40%, then the effect of the particle bodies or the like in improving the contact efficiency between the treatment target liquid and the skeleton interior and skeleton surface of the monolithic ion exchanger may sometimes diminish. Examples of the method used for measuring the coverage rate of the particle bodies or the like include image analysis methods using an SEM image of the monolithic ion exchanger No. 5.

The total pore volume per unit weight in a dry state of the monolithic ion exchanger No. 5 is, for example, within a range from 0.5 to 10 mL/g, and preferably within a range from 0.8 to 8 mL/g. If the total pore volume is less than 0.5 mL/g, then when the ion exchanger is packed into a column and a treatment target liquid is passed through the column, the pressure loss during liquid passage may sometimes increase, and moreover, the transmitted fluid volume per unit cross-sectional area may decrease, causing a fall in the processing capacity. If the total pore volume exceeds 10 mL/g, then the mechanical strength decreases, and when the ion exchanger is packed in a column and a treatment target liquid is passed through the column, the monolithic ion exchanger may sometimes deform, particularly when the liquid is passed through at a high flow rate. Moreover, the contact efficiency between the treatment target liquid and the monolithic ion exchanger may sometimes deteriorate.

In the monolithic ion exchanger No. 5, the crosslinking density of the polymer material that constitutes the skeleton is such that, relative to all of the structural units that constitute the polymer material, the number of crosslinked structural units is, for example, within a range from 0.3 to 10 mol%, and preferably within a range from 0.3 to 5 mol%. The organic polymer material that constitutes the skeleton of the monolithic ion exchanger No. 5 is the same as that of the monolithic ion exchanger No. 1.

In the monolithic ion exchanger No. 5, the material that constitutes the skeleton phase of the organic porous body and the particle bodies or the like formed on the surface of the skeleton phase may be of the same material and be a continuation of the same structure, or may be of different materials, so that materials that are not the same form a continuous structure. Examples of the cases where different materials that are not the same form a continuous structure include those cases where materials are formed using different vinyl monomers, or those cases where although the materials employ the same vinyl monomer and crosslinking agent, the blend ratio between those components differs.

The monolithic ion exchanger No. 5 has a thickness, for example, of 1 mm or greater, and can be distinguished from a film-like porous body. The thickness of the monolithic ion exchanger No. 5 is preferably within a range from 3 to 1,000 mm.

The ion exchange capacity per unit weight in a dry state is as described above. Further, the fact that "the ion exchange groups are distributed throughout the organic porous ion exchanger" is also as described above.

### <Method for Producing Monolithic Ion Exchanger No. 5>

The monolithic ion exchanger No. 5 can be produced, for example, using the method described below.

The monolithic ion exchanger No. 5 can be obtained, for example, by preparing a water-in-oil emulsion, and then conducting steps I to III described below. In step I, for example, the water-in-oil emulsion is polymerized to obtain a monolithic organic porous intermediate (hereinafter also referred to as the monolith intermediate (5)) with a continuous macroporous structure having a total pore volume, for example, within a range from 5 to 30 mL/g. In step II, a mixture is prepared containing, for example, a vinyl monomer, a crosslinking agent having at least two vinyl groups within each molecule, an organic solvent that dissolves the vinyl monomer and the crosslinking agent but does not dissolve the polymer produced by polymerization of the vinyl monomer, and a polymerization initiator. In step III, for example, the mixture obtained in step II is subjected to polymerization in a stationary state and in the presence of the monolith intermediate (5) obtained in step I, thus obtaining a monolith No. 5.

Step I in the method for producing the monolith No. 5 is the same as step I in the method for producing the monolith No. 3.

In step I, during the formation of the water-in-oil emulsion, a polymerization initiator may also be used if necessary. The polymerization initiator is preferably a compound that generates radicals upon heating or light irradiation. The polymerization initiator may be either water-soluble or oil-soluble, and examples include 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobisisobutyrate, 4,4'-azobis(4-cyanovaleric acid), 1,1'-azobis(cyclohexane-1-carbonitrile), benzoyl peroxide, lauroyl peroxide, potassium persulfate, ammonium persulfate, hydrogen peroxide-ferrous chloride, and sodium persulfate-sodium hydrogen sulfite.

The monolith intermediate (5) obtained in step I has a continuous macroporous structure. By including this intermediate in the polymerization system, particle bodies or the like are formed on the surface of the skeleton phase of the continuous macroporous structure, or particle bodies or the like are formed on the surface of the skeleton phase of the co-continuous structure, with the structure of the monolith intermediate (5) acting as a matrix. Further, the monolith intermediate (5) is an organic polymer material having a crosslinked structure. The crosslinking density of the polymer material is such that, relative to all of the structural units that constitute the polymer material, the number of crosslinked structural units is, for example, within a range from 0.3 to 10 mol%, and preferably within a range from 0.3 to 5 mol%.

The types of polymer material for the monolith intermediate (5) are the same as the types of polymer material for the monolith intermediate (3) in the method for producing the monolith No.3.

The total pore volume per unit weight in a dry state of the monolith intermediate (5) obtained in step I is, for example, within a range from 5 to 30 mL/g, and preferably within a range from 6 to 28 mL/g. In order to ensure that the total pore volume of the monolith intermediate (5) falls within the above numerical range, the ratio (weight ratio) between the monomer and water is, for example, within a range from about 1:5 to 1:35.

In step I, if this ratio between the monomer and water is within a range from about 1:5 to 1:20, then a monolith intermediate (5) with a continuous macroporous structure having a total pore volume, for example, within a range from 5 to 16 mL/g is obtained, and the monolith obtained following the completion of step III becomes a monolith No. 5-1. On the other hand, if this ratio between the monomer and water is within a range from about 1:20 to 1:35, then a monolith intermediate (5) is obtained with a continuous macroporous structure having a total pore volume that, for example, exceeds 16 mL/g but is not more than 30 mL/g, and the monolith obtained following the completion of step III becomes a monolith No. 5-2.

In the monolith intermediate (5) obtained in step I of the method for producing the monolith No. 5, the average diameter in a dry state of the openings (mesopores) that represent the overlapping portions between macropores is, for example, within a range from 20 to 200 µm.

Step II in the method for producing the monolith No. 5 is the same as step II in the method for producing the monolith No. 3. In step III in the method for producing the monolith No. 5, for example, the mixture obtained in step II is subjected to polymerization in a stationary state and in the presence of the monolith intermediate (5) obtained in step I, thus obtaining a monolith No. 5.

Here, as disclosed in JP H07-501140 A, by subjecting the vinyl monomer and the crosslinking agent to stationary polymerization in a specific organic solvent in the absence of the monolith intermediate (5), a particle aggregate-type monolithic organic porous body can be obtained. In contrast, by including the monolith intermediate (5) with the continuous macroporous structure in the above polymerization system, the structure of the composite monolith following polymerization is dramatically changed, and rather than a particle aggregate-type structure, the monolith No. 5 having the specific skeleton structure described above can be obtained.

In step III in the method for producing the monolith No. 5, there are no particular limitations on the internal capacity of the reaction container, provided it is sufficiently large to enable the monolith intermediate (5) to be included in the reaction container. The size of the container may be such that when the monolith intermediate (5) is placed inside the reaction container and then viewed in plan view, there are still gaps around the periphery of the monolith, or may be such that the monolith intermediate (5) fills the inside of the reaction container with almost no gaps. Of these possibilities, the case where the monolith No. 5 following polymerization is exposed to almost no pressure from the internal walls of the container but fills the reaction container with almost no gaps is efficient in terms of causing almost no deformation of the monolith No. 5 while ensuring almost no wastage of the reaction raw materials and the like. Even in those cases where the internal capacity of the reaction container is large and there are gaps around the periphery of the monolith No. 5 following polymerization, the vinyl monomer and the crosslinking agent still adsorb to and are distributed across the monolith intermediate (5), meaning there is almost no formation of particle aggregate-type structures in the gaps inside the reaction container.

In this step III, for example, the monolith intermediate (5) is placed in a reaction container in a state impregnated with the mixture (solution). The blend ratio between the mixture obtained in step II and the monolith intermediate (5) is adjusted so that, as described above, the amount added of the vinyl monomer, expressed as a weight, is within a range from 3 to 50 times, and preferably within a range from 4 to 40 times, the weight of the monolith intermediate (5). As a result, a monolith No. 5 can be obtained that is a composite monolith having an appropriate diameter for the openings and having a specific skeleton. In the reaction container, the vinyl monomer and the crosslinking agent in the mixture adsorb to and are distributed across the skeleton of the stationary monolith intermediate (5), with the polymerization then progressing within the skeleton of the monolith intermediate (5).

In step III in the method for producing the monolith No. 5, the polymerization conditions may be substantially the same as those in step III in the method for producing the monolith No. 3.

When producing the monolith No. 5 described above, if step II or step III is conducted under conditions that satisfy at least one of the following conditions (1) to (5), then a monolith having particle bodies or the like formed on the skeleton surface can be produced.
(1) The polymerization temperature in step III is a temperature at least 5°C lower than the 10-hour half-life temperature of the polymerization initiator.
(2) The mol% of the crosslinking agent used in step II is at least two times the mol% of the crosslinking agent used in step I.
(3) The vinyl monomer used in step II is a vinyl monomer having a different structure from that of the oil-soluble monomer used in step I.
(4) The organic solvent used in step II is a polyether with a molecular weight of 200 or greater.
(5) The concentration of the vinyl monomer used in step II is not more than 30% by weight of the mixture in step II.

Examples of preferred structures for the monolith No. 5 obtained in this manner include a continuous macroporous structure in which bubble-shaped macropores overlap with each other, with these overlapping portions forming openings having an average diameter in a dry state, for example, within a range from 10 to 120 µm (the monolith No. 5-1), and a co-continuous structure composed of a three-dimensionally continuous skeleton in which the thickness of the continuous skeleton in a dry state is, for example, within a range from 0.8 to 40 µm, and three-dimensionally continuous pores within that skeleton, with the pores having an average diameter in a dry state that is, for example, within a range from 8 to 80 µm (the monolith No. 5-2). The method used for introducing ion exchange groups into the monolith No. 5 may be the same as the methods used for introducing ion exchange groups into the monolith No. 1.

### <Ion Exchange Methods using the Ion Exchangers>

Ion exchange (anion exchange) can be conducted using the ion exchangers according to embodiments of the present invention. The ion exchange may be conducted by bringing a treatment target liquid and the ion exchanger into contact, for example, at a temperature within a range from 0 to 100°C. For example, the ion exchange may be conducted by passing the treatment target liquid through a column packed with the ion exchanger at a temperature of 0 to 100°C.

The ion exchange method using an ion exchanger according to an embodiment of the present invention may be used, for example, in the purification of water, sugar solutions, and organic solvents and the like, in synthetic processes for chemical products such as pharmaceutical intermediates, or as a filler for ion chromatography used for analysis purposes.

By using the ion exchanger according to an embodiment of the present invention, even if an organic solvent is passed through a column packed with the ion exchanger in a wet state, there is little shrinkage of the ion exchanger, and the organic solvent can be efficiently brought into contact with the ion exchanger.

### [Catalyst having a Platinum group Metal Ion Supported thereon]

The catalyst having a platinum group metal ion supported thereon according to an embodiment of the present invention is a catalyst in which at least one of a platinum group metal ion and a platinum group metal complex ion is supported on the ion exchanger described above. In other words, in the catalyst having a platinum group metal ion supported thereon, the platinum group metal is supported in an ionic form on the above ion exchanger. For example, the catalyst having a platinum group metal ion supported thereon may have the platinum group metal ion or platinum group metal complex ion supported via ionic bonding or coordinate bonding to quaternary ammonium groups within the ion exchanger. The catalyst having a platinum group metal ion supported thereon according to an embodiment of the present invention is preferably a catalyst in which at least one of a platinum group metal ion and a platinum group metal complex ion is supported on a non-particulate organic porous ion exchanger described above, for example, any one of the monolithic ion exchanger No. 1 through to the monolithic ion exchanger No. 5.

The platinum group metals are ruthenium, rhodium, palladium, osmium, iridium and platinum. Platinum group metal ions are ions of one of these platinum group metals, with the valency of the platinum group metal ion varying depending on the type of platinum group metal. One type of these platinum group metal ions may be used alone, or a combination of ions of two or more metals may be used. Among the various possibilities, platinum ions and palladium ions are preferred in terms of increasing the catalytic activity.

Platinum group metal complex ions are complex ions of the above platinum group metals, and examples include palladium complex ions, platinum complex ions, and iridium complex ions. One type of these platinum group metal complex ions may be used alone, or a combination of complex ions of two or more metals may be used. Among the various possibilities, platinum complex ions and palladium complex ions are preferred in terms of increasing the catalytic activity.

In the catalyst having a platinum group metal ion supported thereon, confirmation that platinum group metal ions or platinum group metal complex ions are supported on the catalyst can be made by transmission electron microscope (TEM) observation.

The amount of supported platinum group metal ions or platinum group metal complex ions within the catalyst having a platinum group metal ion supported thereon ((equivalent mass of platinum group metal atoms / mass of catalyst having a platinum group metal ion supported thereon in a dry state) × 100) is, for example, an atom-equivalent amount within a range from 0.01 to 10% by mass, and preferably within a range from 0.1 to 5.0% by mass. By ensuring that the supported amount of platinum group metal ions or platinum group metal complex ions falls within this range, the catalyst can function more readily as a catalyst for carbon-carbon bond forming reactions, and the raw materials are less expensive. Quantification of the platinum group metal atoms within the catalyst having a platinum group metal ion supported thereon can be conducted using an ICP emission spectrometer.

There are no particular limitations on the method used for producing the catalyst having a platinum group metal ion supported thereon, and the catalyst having a platinum group metal ion supported thereon can be obtained by using a conventional method to support at least one of platinum group metal ions and platinum group metal complex ions on the ion exchanger. Examples include methods in which an aforementioned monolithic ion exchanger in a dry state is immersed in an organic solvent of a platinum group metal compound at a prescribed temperature for a prescribed period of time, thereby adsorbing platinum group metal ions to the monolithic ion exchanger by ion exchange, and methods in which the monolithic ion exchanger is immersed in an aqueous solution of a platinum group metal complex compound such as a tetraammine palladium complex at a prescribed temperature for a prescribed period of time, thereby adsorbing and supporting platinum group metal ions on the monolithic ion exchanger by ion exchange.

Supporting the platinum group metal ions or platinum group metal complex ions on the monolithic ion exchanger may be conducted in a batch system or a flow system, and there are no particular limitations.

The platinum group metal compound used in the method for producing the catalyst having a platinum group metal ion supported thereon may be either an organic salt or an inorganic salt, and examples include halides, sulfates, nitrates, phosphates, organic acid salts, and inorganic complex salts. Specific examples of the platinum group metal compound include palladium chloride, palladium nitrate, palladium sulfate, palladium acetate, tetraammine palladium chloride, tetraammine palladium nitrate, platinum chloride, tetraammine platinum chloride, tetraammine platinum nitrate, chlorotriammine platinum chloride, hexaammine platinum chloride, hexaammine platinum sulfate, chloropentaammine platinum chloride, cis-tetrachlorodiammine platinum chloride, trans-tetrachlorodiammine platinum chloride, rhodium chloride, rhodium acetate, hexaammine rhodium chloride, hexaammine rhodium bromide, hexaammine rhodium sulfate, pentaammine aqua rhodium chloride, pentaammine aqua rhodium nitrate, cis-dichlorotetraammine rhodium chloride, trans-dichlorotetraammine rhodium chloride, ruthenium chloride, hexaammine ruthenium chloride, hexaammine ruthenium bromide, hexaammine ruthenium iodide, chloropentaammine ruthenium chloride, cis-dichlorotetraammine ruthenium chloride, trans-dichlorotetraammine rhodium chloride, iridium (III) chloride, iridium (IV) chloride, hexaammine iridium chloride, hexaammine iridium nitrate, chloropentaammine iridium chloride, chloropentaammine iridium bromide, hexaammine osmium chloride, hexaammine osmium bromide, and hexaammine osmium iodide. The amounts used of these compounds, for example, expressed as a metal-equivalent weight relative to the weight of the monolithic ion exchanger that acts as the support, is typically within a range from 0.005 to 30% by weight.

When supporting the platinum group metal ions or platinum group metal complex ions, the platinum group metal compound is typically dissolved in a solvent prior to use. Examples of the solvent include water; alcohols such as methanol, ethanol, propanol, butanol, and benzyl alcohol; ketones such as acetone and methyl ethyl ketone; nitriles such as acetonitrile; amides such as dimethylformamide, dimethylacetamide and N-methylpyrrolidone; and mixtures of these solvents. Further, in order to enhance the solubility of the platinum group metal compound in the solvent, an acid such as hydrochloric acid, sulfuric acid or nitric acid, or a base such as sodium hydroxide or tetramethylammonium hydroxide may also be added.

### <Method for Forming Carbon-Carbon Bond>

The method for forming a carbon-carbon bond according to an embodiment of the present invention is a method for forming a carbon-carbon bond, for example, by conducting, in the presence of the catalyst having a platinum group metal ion supported thereon described above, (1) a reaction between an aromatic halide and an organic boron compound, (2) a reaction between an aromatic halide and a compound having an alkynyl group at a terminal, or (3) a reaction between an aromatic halide and a compound having an alkenyl group.

A first configuration of the method for forming a carbon-carbon bond (hereinafter also referred to as the carbon-carbon bond forming method (1)) is a reaction for generating a carbon-carbon single bond by reacting and coupling an aromatic halide and an organic boron compound in the presence of the catalyst having a platinum group metal ion supported thereon described above.

The organic boron compound used in the carbon-carbon bond forming method (1) is an organic boron compound represented by the following formula:

R⁴-B(OH)₂

(wherein R⁴ represents an organic group, and although any organic group may be used without any particular limitations, examples include linear alkyl groups, branched alkyl groups, cyclic alkyl groups, aromatic carbocyclic groups and aromatic heterocyclic groups, and provided the effects of the present embodiment are not impaired, these groups may also include an introduced methyl group, ethyl group, nitro group, amino group, methoxy group, ethoxy group, carboxymethyl group, or acetyl group or the like).

The organic boron compound used in the carbon-carbon bond forming method (1) is, for example, an aromatic boron compound represented by general formula (I) shown below:

Ar¹-B(OH)₂ (I)

(wherein Ar¹ represents an aromatic carbocyclic group or aromatic heterocyclic group of 6 to 18 carbon atoms).

In formula (I), examples of the aromatic carbocyclic group or aromatic heterocyclic group for Ar¹ include a phenyl group, naphthyl group, biphenyl group, anthranyl group, pyridyl group, pyrimidyl group, indolyl group, benzimidazolyl group, quinolyl group, benzofuranyl group, indanyl group, indenyl group and dibenzofuranyl group. There are no particular limitations on the position at which the boron is bonded to the aromatic carbocyclic group or aromatic heterocyclic group, and bonding at any arbitrary position is possible. Further, one or more substituents may be introduced into the aromatic carbocyclic group or aromatic heterocyclic group. Examples of these substituents include hydrocarbon groups such as a methyl group, ethyl group, propyl group, butyl group, hexyl group or benzyl group; alkoxy groups such as a methoxy group, ethoxy group, propoxy group or butoxy group; as well as a 9-fluorenylmethoxycarbonyl group, butoxycarbonyl group, benzyloxycarbonyl group or nitro group.

The aromatic halide used in the carbon-carbon bond forming method (1) is, for example, an aromatic halide represented by general formula (II) shown below:

Ar²-X (II)

(wherein Ar² represents an aromatic carbocyclic group or aromatic heterocyclic group of 6 to 18 carbon atoms, and X represents a halogen atom).

In formula (II), examples of the aromatic carbocyclic group or aromatic heterocyclic group for Ar² include the same groups as Ar¹. There are no particular limitations on the position at which the halogen atom is bonded to the aromatic carbocyclic group or aromatic heterocyclic group, and bonding at any arbitrary position is possible. Further, one or more substituents may be introduced into the aromatic carbocyclic group or aromatic heterocyclic group. Examples of these substituents include hydrocarbon groups such as a methyl group, ethyl group, propyl group, butyl group, hexyl group or benzyl group; alkoxy groups such as a methoxy group, ethoxy group, propoxy group or butoxy group; as well as a 9-fluorenylmethoxycarbonyl group, butoxycarbonyl group, benzyloxycarbonyl group, nitro group, carboxyl group or amino group.

X represents a halogen atom, and specific examples include a fluorine atom, chlorine atom, bromine atom or iodine atom.

Generation of a carbon-carbon bond by the carbon-carbon bond forming method (1) refers to the generation of a carbon-carbon bond between the organic group formed upon elimination of a functional group containing boron from the organic boron compound, and the aromatic residue formed upon elimination of the halogen from the aromatic halide. For example, in the case where the aromatic boron compound is an aromatic boron compound represented by formula (I), and the aromatic halide is an aromatic halide represented by formula (II), the resulting coupled product is a compound represented by formula (III):

Ar¹-Ar² (III)

(wherein Ar¹ and Ar² are the same as described above in formula (I) and formula (II)).

Although there are no particular limitations on the proportions used of the aromatic boron compound and the aromatic halide in the carbon-carbon bond forming method (1), for example, the molar ratio of aromatic boron compound : aromatic halide is typically within a range from (0.5 to 2): 1, and an equimolar ratio may be used.

A second configuration of the method for forming a carbon-carbon bond (hereinafter also referred to as the carbon-carbon bond forming method (2)) is a reaction for forming a carbon-carbon single bond by reacting an aromatic halide and a compound having an alkynyl group at a terminal in the presence of the catalyst having a platinum group metal ion supported thereon described above.

The aromatic halide used in the carbon-carbon bond forming method (2) is, for example, an aromatic halide represented by general formula (II) shown above.

The compound having an alkynyl group at a terminal used in the carbon-carbon bond forming method (2) is, for example, a compound represented by formula (IV) shown below:

HC≡C-R⁵ (IV)

(wherein R⁵ represents a hydrogen atom, an aromatic carbocyclic group of 6 to 18 carbon atoms which may have a substituent, an aromatic heterocyclic group of 6 to 18 carbon atoms which may have a substituent, an aliphatic hydrocarbon group of 1 to 18 carbon atoms which may have a substituent, an alkenyl group of 2 to 18 carbon atoms which may have a substituent, or an alkynyl group of 2 to 10 carbon atoms which may have a substituent).

In formula (IV), examples of the aromatic carbocyclic group of 6 to 18 carbon atoms which may have a substituent or the aromatic heterocyclic group of 6 to 18 carbon atoms which may have a substituent for R⁵ include the same groups as those described for Ar¹ and Ar² in formula (I) and formula (II).

In formula (IV), examples of the aliphatic hydrocarbon group of 1 to 18 carbon atoms which may have a substituent for R⁵ include a methyl group, ethyl group, propyl group, butyl group, hexyl group, octyl group, dodecyl group and octadecyl group.

In formula (IV), examples of the alkenyl group of 2 to 18 carbon atoms which may have a substituent for R⁵ include a vinyl group, allyl group, methallyl group, propenyl group, butenyl group, hexenyl group, octenyl group, decenyl group and octadecenyl group.

In formula (IV), examples of the alkynyl group of 2 to 10 carbon atoms which may have a substituent for R⁵ include an ethynyl group, propynyl group, hexynyl group and octynyl group.

Examples of the substituent which these aromatic carbocyclic groups, aromatic heterocyclic groups, aliphatic hydrocarbon groups, alkenyl groups and alkynyl groups may have include a hydroxyl group, hydrocarbon groups such as a phenyl group, and heteroatom-containing hydrocarbon groups such as a methoxy group or trifluoromethoxy group.

In the carbon-carbon bond forming method (2), for example, an aromatic halide represented by formula (II) is reacted with a compound represented by formula (IV) in the presence of the catalyst having a platinum group metal ion supported thereon described above, yielding a product of formula (V):

Ar²-C≡C-R⁵ (V)

(wherein Ar² and R⁵ are the same as described above in formula (II) and formula (IV)).

Although there are no particular limitations on the proportions used of the aromatic halide and the compound having an alkynyl group at a terminal in the carbon-carbon bond forming method (2), for example, the molar ratio of aromatic halide : compound having an alkynyl group at a terminal is typically within a range from (0.5 to 3): 1, and an equimolar ratio may be used.

A third configuration of the method for forming a carbon-carbon bond (hereinafter also referred to as the carbon-carbon bond forming method (3)) is a reaction for forming a carbon-carbon single bond by reacting an aromatic halide and a compound having an alkenyl group in the presence of the catalyst having a platinum group metal ion supported thereon described above.

The aromatic halide used in the carbon-carbon bond forming method (3) is, for example, an aromatic halide represented by general formula (II) shown above.

The compound having an alkenyl group used in the carbon-carbon bond forming method (3) is, for example, a compound represented by formula (VI):

R⁶HC=CR⁷R⁸ (VI)

(wherein R⁶, R⁷ and R⁸ each independently represent a hydrogen atom, an aromatic carbocyclic group of 6 to 18 carbon atoms which may have a substituent, an aromatic heterocyclic group of 6 to 18 carbon atoms which may have a substituent, an aliphatic hydrocarbon group of 1 to 18 carbon atoms which may have a substituent, a carboxylic acid derivative, an acid amide derivative, or a cyano group).

In formula (VI), examples of the aromatic carbocyclic group of 6 to 18 carbon atoms which may have a substituent, the aromatic heterocyclic group of 6 to 18 carbon atoms which may have a substituent, and the aliphatic hydrocarbon group of 1 to 18 carbon atoms which may have a substituent for R⁶, R⁷ and R⁸ include the same groups as those described for R¹ in formula (IV).

In formula (VI), examples of the carboxylic acid derivative for R⁶, R⁷ and R⁸ include alkoxycarbonyl groups such as a methoxycarbonyl group, ethoxycarbonyl group and butoxycarbonyl group.

In formula (VI), examples of the acid amide derivative for R⁶, R⁷ and R⁸ include carbamoyl groups such as an N-methylcarbamoyl group and an N,N-dimethylcarbamoyl group.

In the carbon-carbon bond forming method (3), an aromatic halide represented by formula (II) is reacted with a compound represented by formula (VI) in the presence of the catalyst having a platinum group metal ion supported thereon described above, yielding a product of formula (VII):

R⁶Ar²C=CR⁷R⁸ (VII)

(wherein Ar², R⁶, R⁷ and R⁸ are the same as described above in formula (II) and formula (VI)).

Although there are no particular limitations on the proportions used of the aromatic halide and the compound having an alkenyl group in the carbon-carbon bond forming method (3), for example, the molar ratio of aromatic halide : compound having an alkenyl group is typically within a range from (0.5 to 2): 1, and an equimolar ratio may be used.

In the carbon-carbon bond forming reactions (1) to (3), the amount used of the catalyst having a platinum group metal ion supported thereon, for example, expressed as an equivalent amount of the platinum group metal relative to the amount of the aromatic halide, is within a range from 0.01 to 20 mol%.

In the method for forming a carbon-carbon bond, the coupling reaction may be conducted using a solvent, or may be conducted without a solvent. Examples of solvents that may be used include water and organic solvents, as well as mixtures thereof. Examples of the organic solvents include alcohols such as methanol, ethanol, propanol, butanol, ethylene glycol and glycerol; and cyclic ethers such as tetrahydrofuran and dioxane.

The atmosphere in which the method for forming a carbon-carbon bond is conducted may by an open air atmosphere, but is preferably an inert gas atmosphere of nitrogen or argon or the like. There are no particular limitations on the reaction temperature, which may be set arbitrarily, for example, within a range from -20°C to 150°C, and there are also no particular limitations on the reaction time, which may be set, for example, within a range from one minute to 24 hours.

In the carbon-carbon bond forming reaction, a base is preferably also included, and including an inorganic base is particularly preferred. Examples of bases that may be used include inorganic bases such as sodium carbonate, sodium bicarbonate, potassium carbonate, cesium carbonate, potassium acetate, sodium phosphate, potassium phosphate and barium hydroxide; and organic bases such as potassium phenolate, sodium methoxide, sodium ethoxide, potassium butoxide, trimethylamine and triethylamine. The amount used of these bases is set, for example, within a range from 50 to 300 mol% relative to the aromatic halide.

In the method for forming a carbon-carbon bond according to an embodiment of the present invention, the reaction may be conducted by passing the reaction raw material liquid through the catalyst having a platinum group metal ion supported thereon to generate the carbon-carbon bond.

In the method for forming a carbon-carbon bond according to an embodiment of the present invention, one of the aforementioned carbon-carbon bond forming reactions (1) to (3) is, for example, preferably conducted by introducing a raw material liquid (i) containing the aromatic halide and the organic boron compound described above, a raw material liquid (ii) containing the aromatic halide and the compound having an alkynyl group at a terminal described above, or a raw material liquid (iii) containing the aromatic halide and the compound having an alkenyl group described above into a packed container packed with a catalyst having a platinum group metal ion supported thereon from an introduction passage of the packed container, passing the raw material liquid through the catalyst having a platinum group metal ion supported thereon, and discharging the reaction liquid from a discharge passage of the packed container.

In the method for forming a carbon-carbon bond, for example, the raw material liquid (i), the raw material liquid (ii) or the raw material liquid (iii) may be a dissolved inorganic base-containing raw material liquid containing the raw materials and an inorganic base dissolved in water or a hydrophilic solvent, and the carbon-carbon bond forming reaction may be conducted by introducing the dissolved inorganic base-containing raw material liquid into a packed container packed with a catalyst having a platinum group metal ion supported thereon from an introduction passage of the packed container, passing the dissolved inorganic base-containing raw material liquid through the catalyst having a platinum group metal ion supported thereon, and then discharging the reaction liquid from a discharge passage of the packed container.

Further, in the method for forming a carbon-carbon bond, for example, the raw material liquid (i), the raw material liquid (ii) or the raw material liquid (iii) may be a hydrophobic solvent raw material liquid containing the raw materials dissolved in a hydrophobic organic solvent, and the carbon-carbon bond forming reaction may be conducted by introducing a mixture of the hydrophobic solvent raw material liquid and an inorganic base aqueous solution containing a dissolved inorganic base into a packed container packed with a catalyst having a platinum group metal ion supported thereon from an introduction passage of the packed container, passing the hydrophobic solvent raw material liquid and the inorganic base aqueous solution through the catalyst having a platinum group metal ion supported thereon, and then discharging the reaction liquid from a discharge passage of the packed container.

The method for forming a carbon-carbon bond according to an embodiment of the present invention forms a carbon-carbon bond to produce a desired compound, and enables the target product to be obtained at high yield. In particular, the carbon-carbon bond forming reaction can be conducted with high yield even with an aromatic bromide. Further, the target product can be obtained in high yield with a short reaction time. By using the method for forming a carbon-carbon bond according to an embodiment of the present invention, the method for forming a carbon-carbon bond used for forming the carbon-carbon bond and obtaining the desired compound can be conducted in a fixed bed continuous flow system, and the carbon-carbon bond forming reaction can be conducted with high yield using a variety of raw materials. Furthermore, from the viewpoint of production efficiency, a high-concentration raw material solution may also be used.

### EXAMPLES

The present invention is described below in further detail using a series of examples and comparative examples, but the present invention is not limited to the following examples.

### <Example 1>

A monolith was produced in accordance with the method for producing the monolithic ion exchanger No. 5, and ion exchange groups were introduced into the obtained monolith.

### (Production of Monolith Intermediate (Step I))

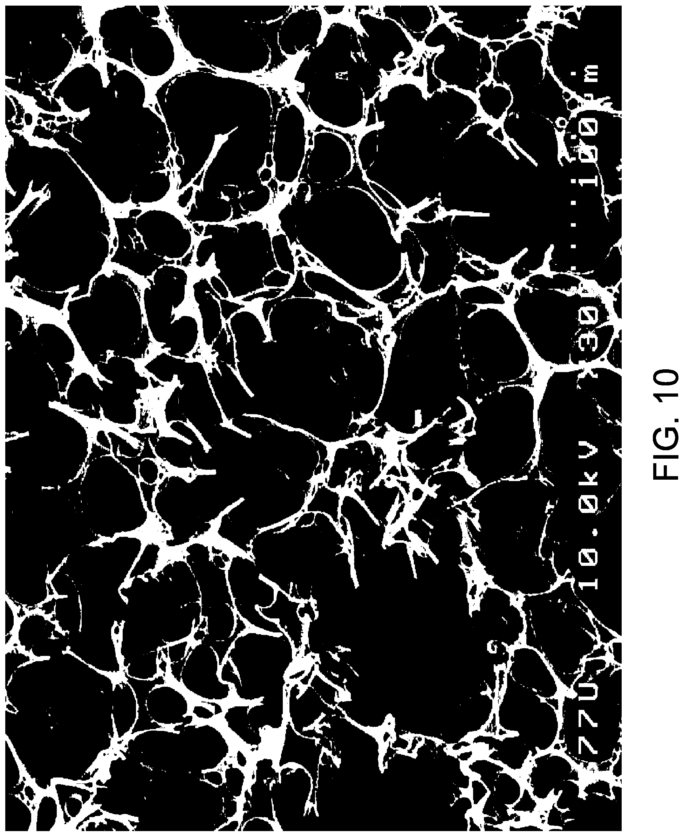

First, 9.28 g of styrene and 0.19 g of divinylbenzene as monomers, 0.50 g of sorbitan monooleate (hereinafter abbreviated as SMO) as a surfactant, and 0.25 g of 2,2'-azobis(isobutyronitrile) as a polymerization initiator were mixed together and dissolved uniformly. Next, this styrene / divinylbenzene / SMO / 2,2'-azobis(isobutyronitrile) mixture was added to 180 g of pure water, the resulting mixture was stirred under reduced pressure using a vacuum stirring and defoaming mixer which was a planetary mixer (manufactured by EME GmbH), thus obtaining a water-in-oil emulsion. This emulsion was promptly transferred to a reaction container, and following sealing, a stationary polymerization was conducted at 60°C for 24 hours. Following completion of the polymerization, the contents were removed, extracted with methanol, and then dried under reduced pressure to complete production of a monolith intermediate having a continuous macroporous structure. The internal structure of the monolith intermediate (dried body) obtained in this manner was inspected using a SEM. The SEM image is shown in FIG. 10, and reveals a continuous macroporous structure even though the wall portions that separate adjacent macropores are extremely thin rod-shaped structures, wherein measurements by mercury porosimetry showed that the average diameter of the openings (mesopores) that represent the overlapping portions between macropores was 40 µm, and the total pore volume was 18.2 mL/g.

### (Production of Monolith (Step II))

Subsequently, 216.6 g of styrene as a monomer, 4.4 g of divinylbenzene as a crosslinking agent, 220 g of 1-decanol as an organic solvent, and 0.8 g of 2,2'-azobis(2,4-dimethylvaleronitrile) as a polymerization initiator were mixed together and dissolved uniformly (step II).

### (Production of Monolith (Step III))

Next, the monolith intermediate described above was placed in a reaction container and immersed in the styrene / divinylbenzene / 1-decanol / 2,2'-azobis(2,4-dimethylvaleronitrile) mixture, and following defoaming in a reduced-pressure chamber, the reaction container was sealed, and a stationary polymerization was conducted at 50°C for 24 hours. Following completion of the polymerization, the contents were removed, a Soxhlet extraction was conducted with acetone, and the product was then dried under reduced pressure (step III).

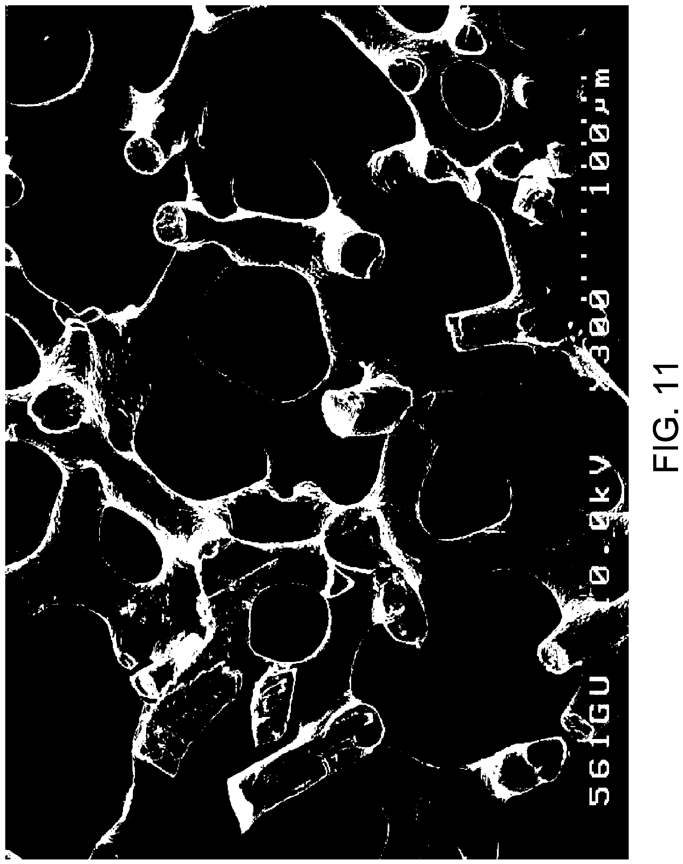

The result of SEM observation of the internal structure of the monolith (dried product) containing 1.2 mol% of a crosslinked component formed from the styrene/divinylbenzene-based copolymer obtained in this manner is shown in FIG. 11. As is evident from FIG. 11, this monolith was a co-continuous structure composed of a continuous skeleton phase and a continuous pore phase in which both the skeleton and the pores were three-dimensionally continuous and these two phases were intertwined. Further, the thickness of the continuous skeleton measured from the SEM image was 20 µm. Furthermore, measurements by mercury porosimetry revealed that the average diameter of the three-dimensionally continuous pores of this monolith was 70 µm, and the total pore volume was 4.4 mL/g. The average diameter of the pores was determined from the value at the maximum of the pore distribution curve obtained by mercury porosimetry.

### (Production of Chloromethylated Monolith)

The produced monolith was placed in a column-shaped reactor, a solution containing 1,600 g of chlorosulfonic acid, 400 g of tin tetrachloride and 2,500 mL of dimethoxymethane was circulated through the reactor, and this reaction was continued at 30°C for 5 hours to introduce chloromethyl groups. Following completion of the reaction, the chloromethylated monolith was washed using a mixed solvent of THF/water = 2/1 (vol), and then further washed with THF, yielding a chloromethylated monolith.

### (Production of Monolithic Ion Exchanger)

First, 390 mL of THF and 30 mL of N,N-dimethylbenzylamine as a tertiary amine were added to 20 g of the chloromethylated monolith, and the resulting mixture was reacted at 60°C for 6 hours. Following completion of the reaction, the product was washed with methanol, and then washed further with pure water, yielding a monolithic ion exchanger composed of a polymer chain represented by formula (4) shown below: 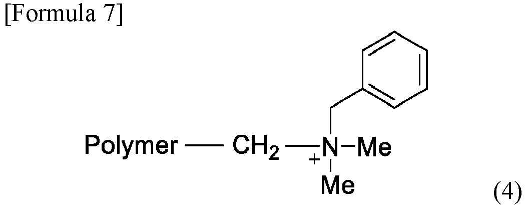 (wherein "Polymer" represents a styrene-divinylbenzene copolymer).

The anion exchange capacity of the obtained monolithic ion exchanger in a dry state was 2.9 mg equivalent/g, confirming that quaternary ammonium groups had been introduced quantitatively. As is evident from FIG. 11, the monolithic anion exchanger was a co-continuous structure in which the skeleton and pores were both three-dimensionally continuous, and these two phases were intertwined. Further, the thickness of the skeleton in a dry state measured from a SEM image was 20 µm, and measurements by mercury porosimetry revealed that the average diameter of the three-dimensionally continuous pores of this monolithic anion exchanger in a dry state was 70 µm, and the total pore volume in a dry state was 4.4 mL/g.

In the following description, the monolithic ion exchanger obtained in Example 1 is referred to as the "benzyl-type monolithic anion exchanger".

### <Comparative Example 1>

With the exception of using a 30% aqueous solution of trimethylamine instead of the N,N-dimethylbenzylamine as the tertiary amine, the same procedure as Example 1 was used to obtain a monolithic ion exchanger composed of a polymer chain represented by formula (5) shown below: 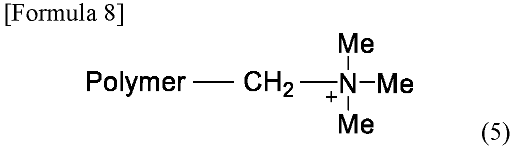 (wherein "Polymer" represents a styrene-divinylbenzene copolymer).

In the following description, the monolithic ion exchanger obtained in Comparative Example 1 is referred to as the "trimethyl-type monolithic anion exchanger".

### <Example 2>

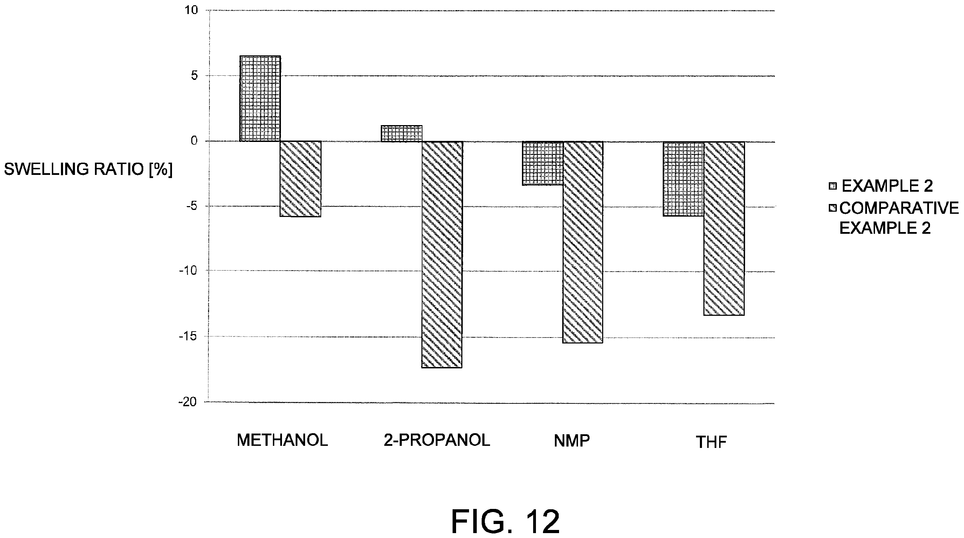

The benzyl-type monolithic anion exchanger obtained in Example 1 was cut to dimensions of 600 mm × 150 mm × 100 mm. This cut sample was immersed in 300 mL of water at room temperature (25±2°C) for 30 minutes to generate a water-wet state, the anion exchanger was then washed three times with 150 mL portions of either methanol, 2-propanol, N-methyl-2-pyrrolidone (NMP) or tetrahydrofuran (THF) as an organic solvent to thoroughly replace the moisture, and the size of the anion exchanger was then measured and the swelling ratio (%) (length ratio) from the water-wet state was estimated. The results are shown in FIG. 12.

### <Comparative Example 2>

With the exception of replacing the benzyl-type monolithic anion exchanger with the trimethyl-type monolithic anion exchanger, the same procedure as Example 2 was conducted. The results are shown in FIG. 12.

As illustrated, the ion exchanger of the example exhibited less shrinkage upon contact with organic solvents in a water-wet state than the ion exchanger of the comparative example.

### <Example 3>

### (Production of Catalyst having a Platinum group Metal Ion Supported thereon)

The produced monolithic ion exchanger was dried under reduced pressure. The weight of the dried monolithic ion exchanger was 8.7 g. This dried monolithic ion exchanger was immersed for 24 hours at 25°C in dilute hydrochloric acid containing 146 mg of dissolved palladium chloride, thereby supporting tetrachloropalladate ions on the ion exchanger. Following completion of the immersion process, the monolithic ion exchanger was washed multiple times with pure water to complete preparation of a Pd ion supported monolithic ion exchanger. Determination of the amount of palladium supported on the obtained Pd ion supported monolithic ion exchanger using an ICP emission spectrometer (model PS3520UVDDII manufactured by Hitachi High-Tech Science Corporation) revealed a supported palladium amount of 1.0% by weight.

In the following description, the catalyst having a platinum group metal ion supported thereon obtained in Example 3 is referred to as the "Pd ion supported monolithic ion exchanger".

### <Example 4>

### (Carbon-Carbon Bond Forming Reactions using Pd Ion Supported Monolithic Ion Exchanger)

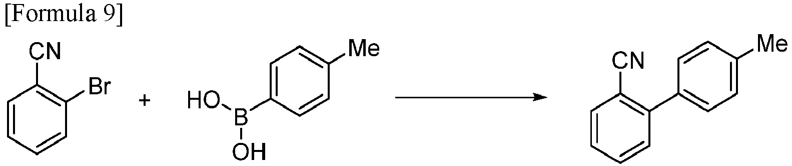

A water (22 mL) solution of sodium hydroxide (35.2 mmol) as an inorganic base was added and stirred into a 4-methyltetrahydropyran (4-MTHP) / ethanol solution (32 mL, 1: 1 (vol)) containing 2-bromobenzonitrile (5.82 g, 32.0 mmol) as an aromatic halide and 4-methylphenylboronic acid (4.78 g, 35.2 mmol) as an organic boron compound. This solution was then passed through an SUS column heated to 80°C and packed with the Pd ion supported monolithic ion exchanger (ø4.6 × 30 mm) at a flow rate of 0.3 mL/minute, and was collected in a flask containing a saturated aqueous solution of ammonium chloride. Analysis of the organic layer of the thus obtained liquid by GC (model GC-2010 manufactured by Shimadzu Corporation) revealed that 2-cyano-4'-methylbiphenyl had been obtained at a conversion rate of 80%.

### <Example 5>

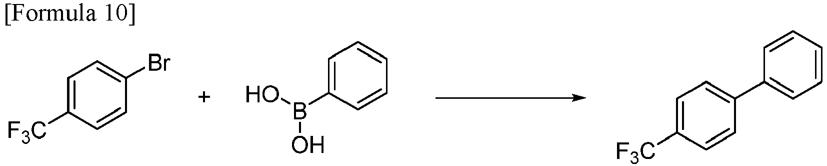

A water (33 mL) solution of sodium hydroxide (52.8 mmol) as an inorganic base was added and stirred into a 4-MTHP/ethanol solution (48 mL, 1: 1 (vol)) containing 4-bromobenzotrifluoride (10.8 g, 48.0 mmol) as an aromatic halide and phenylboronic acid (6.44 g, 52.8 mmol) as an organic boron compound. This solution was then passed through an SUS column heated to 80°C and packed with the Pd ion supported monolithic ion exchanger (ø4.6 × 30 mm) at a flow rate of 0.3 mL/minute, and was collected in a flask containing a saturated aqueous solution of ammonium chloride. Analysis of the organic layer of the thus obtained liquid by GC revealed that 4-(trifluoromethyl)biphenyl had been obtained at a conversion rate of 71%.

### <Example 6>

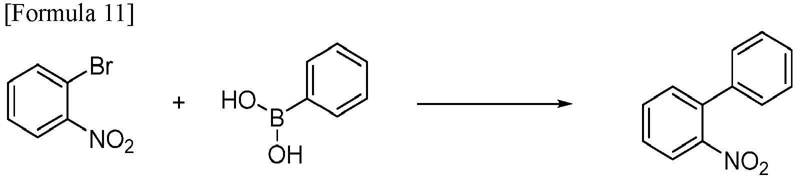

A water (22 mL) solution of sodium hydroxide (35.2 mmol) as an inorganic base was added and stirred into a 4-MTHP/ethanol solution (32 mL, 1: 1 (vol)) containing 2-bromonitrobenzene (6.46 g, 32.0 mmol) as an aromatic halide and phenylboronic acid (4.29 g, 35.2 mmol) as an organic boron compound. This solution was then passed through an SUS column heated to 80°C and packed with the Pd ion supported monolithic ion exchanger (ø4.6 × 30 mm) at a flow rate of 0.3 mL/minute, and was collected in a flask containing a saturated aqueous solution of ammonium chloride. Analysis of the organic layer of the thus obtained liquid by GC revealed that 2-nitrobiphenyl had been obtained at a conversion rate of 81%.

### <Comparative Example 3>

First, 400 mL of THF and 56 mL of a 30% aqueous solution of trimethylamine as a tertiary amine were added to 25.1 g of the chloromethylated monolith obtained in Example 3, and the resulting mixture was reacted at 40°C for 3 hours. Following completion of the reaction, the product was washed with methanol and then with pure water, yielding a monolithic strongly basic anion exchanger composed of a polymer chain represented by formula (5) shown below: 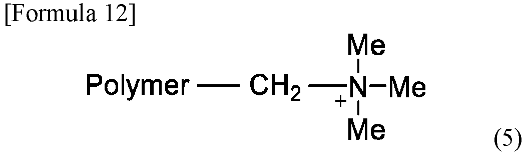 (wherein "Polymer" represents a styrene-divinylbenzene copolymer).

The anion exchange capacity of the obtained monolithic anion exchanger in a dry state was 4.6 mg equivalent/g, confirming that the quaternary ammonium groups had been introduced quantitatively. Further, the thickness of the skeleton in a dry state measured from a SEM image was 20 µm, and measurements by mercury porosimetry revealed that the average diameter of the three-dimensionally continuous pores of this monolithic anion exchanger in a dry state was 70 µm, and the total pore volume in a dry state was 4.4 mL/g.

The obtained monolithic strongly basic anion exchanger was dried under reduced pressure. The weight of the dried monolithic strongly basic anion exchanger was 18.0 g. This dried monolith was immersed for 24 hours at 25°C in dilute hydrochloric acid containing 1.80 g of dissolved palladium chloride, thereby supporting tetrachloropalladate ions on the ion exchanger. Following completion of the immersion process, the monolithic anion exchanger was washed multiple times with pure water to complete preparation of a Pd ion supported monolithic ion exchanger. Determination of the amount of palladium supported on the obtained Pd ion supported monolithic ion exchanger using an ICP emission spectrometer revealed a supported palladium amount of 4.8% by weight.

In the following description, the catalyst having a platinum group metal ion supported thereon obtained in Comparative Example 3 is referred to as the "Pd ion supported monolithic strongly basic anion exchanger".

### <Comparative Example 4>

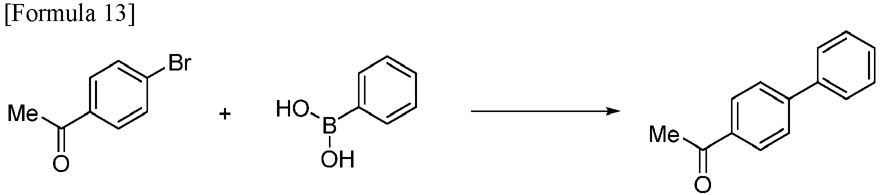

A toluene (2.0 mL) solution containing 4-bromoacetophenone (0.796 g, 4.0 mmol) as an aromatic halide, and a water (4.0 mL) solution containing phenylboronic acid (0.536 g, 4.4 mmol) as an organic boron compound and sodium hydroxide (4.4 mmol) as an inorganic base were fed through separate lines at flow rates of 0.1 mL/minute and 0.2 mL/minute, and were combined and mixed at a joint. The combined liquid was passed through an SUS column heated to 80°C and packed with the Pd ion supported monolithic strongly basic anion exchanger produced in Comparative Example 3 (ø4.6 × 30 mm), and was collected in a flask containing a saturated aqueous solution of ammonium chloride. Analysis of the organic layer of the thus obtained liquid by GC revealed that 4-acetylbiphenyl had been obtained at a conversion rate of 33%.

### <Comparative Example 5>

With the exception of replacing the Pd ion supported monolithic ion exchanger with the Pd ion supported monolithic strongly basic anion exchanger, the same procedure as Example 4 was conducted. The results revealed that 2-cyano-4'-methylbiphenyl had been obtained at a conversion rate of 14%.

In this manner, by using the catalyst having a platinum group metal ion supported thereon of the example, a carbon-carbon bond forming reaction was able to be conducted with high yield even with an aromatic bromide.

### REFERENCE SIGNS LIST

1: Skeleton phase
2: Pore phase
10: Co-continuous structure
11: Rectangular image region
12: Skeleton portion
13: Macropore
21: Skeleton surface
22a, 22b, 22c, 22d, 22e: Protrusion

## Claims

1. An ion exchanger composed of a polymer chain represented by general formula (1): 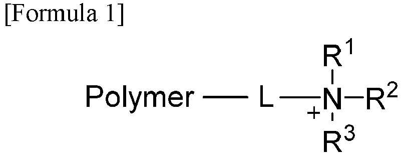 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; R² and R³ each independently represent an alkyl group of 1 to 4 carbon atoms; L represents a linker site; and "Polymer" represents a polymer chain).

2. The ion exchanger according to Claim 1, wherein
L in general formula (1) is a methylene group.

3. The ion exchanger according to Claim 1 or 2, wherein
the polymer chain of the ion exchanger is a styrene-divinylbenzene-based copolymer.

4. The ion exchanger according to any one of Claims 1 to 3, wherein
the ion exchanger is a non-particulate organic porous ion exchanger composed of a continuous skeleton phase and a continuous pore phase, wherein a thickness of the continuous skeleton is within a range from 1 to 100 µm, an average diameter of the continuous pores is within a range from 1 to 1,000 µm, a total pore volume is within a range from 0.5 to 50 mL/g, an ion exchange capacity per unit weight in a dry state is within a range from 1 to 9 mg equivalent/g, and ion exchange groups are distributed throughout the organic porous ion exchanger.

5. A method for producing an ion exchanger by reacting:
a polymer chain represented by general formula (2):
[Formula 2] Polymer - L - X
(wherein L represents a linker site, "Polymer" represents a polymer chain, and X represents a halogen atom, an alkylsulfonyl group of 1 to 8 carbon atoms which may be substituted, or a benzenesulfonyl group which may be substituted), and
a tertiary amine represented by general formula (3): 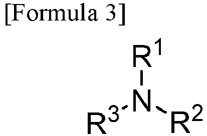 (wherein R¹ represents an alkyl group of 4 to 22 carbon atoms which may be substituted; or a benzyl group which may be substituted with an alkyl group of 1 to 6 carbon atoms which may be substituted, a halogen atom, an alkoxy group of 1 to 6 carbon atoms which may be substituted, an amino group which may be substituted, a cyano group, or a nitro group; and R² and R³ each independently represent an alkyl group of 1 to 4 carbon atoms).

6. The method for producing an ion exchanger according to Claim 5, wherein
L in general formula (1) is a methylene group.

7. The method for producing an ion exchanger according to Claim 5 or 6, wherein
the polymer chain of the ion exchanger is a styrene-divinylbenzene-based copolymer.

8. The method for producing an ion exchanger according to any one of Claims 5 to 7, wherein
the ion exchanger is a non-particulate organic porous ion exchanger composed of a continuous skeleton phase and a continuous pore phase, wherein a thickness of the continuous skeleton is within a range from 1 to 100 µm, an average diameter of the continuous pores is within a range from 1 to 1,000 µm, a total pore volume is within a range from 0.5 to 50 mL/g, an ion exchange capacity per unit weight in a dry state is within a range from 1 to 9 mg equivalent/g, and ion exchange groups are distributed throughout the organic porous ion exchanger.

9. A catalyst having a platinum group metal ion supported thereon, wherein at least one of a platinum group metal ion and a platinum group metal complex ion are supported on the ion exchanger according to any one of Claims 1 to 4.

10. The catalyst having a platinum group metal ion supported thereon according to Claim 9, wherein
an amount supported of at least one of the platinum group metal ion and the platinum group metal complex ion, expressed as an equivalent mass of platinum group metal atoms, is within a range from 0.01 to 10.0% by mass.

11. The catalyst having a platinum group metal ion supported thereon according to Claim 9 or 10, wherein
R¹ in general formula (1) is an alkyl group of 6 to 20 carbon atoms, or a benzyl group which may be substituted with an alkyl group of 1 to 4 carbon atoms.

12. The catalyst having a platinum group metal ion supported thereon according to any one of Claims 9 to 11, wherein
R¹ in general formula (1) is a dodecyl group or a benzyl group.

13. The catalyst having a platinum group metal ion supported thereon according to any one of Claims 9 to 12, wherein
R² and R³ in general formula (1) each independently represent a methyl group or an ethyl group.

14. A method for forming a carbon-carbon bond in which a carbon-carbon bond is formed by conducting (1) a reaction between an aromatic halide and an organic boron compound, (2) a reaction between an aromatic halide and a compound having an alkynyl group at a terminal, or (3) a reaction between an aromatic halide and a compound having an alkenyl group, wherein
a formation reaction for the carbon-carbon bond is conducted by introducing a raw material liquid (i) containing the aromatic halide and the organic boron compound, a raw material liquid (ii) containing the aromatic halide and the compound having an alkynyl group at a terminal, or a raw material liquid (iii) containing the aromatic halide and the compound having an alkenyl group into a packed container packed with a catalyst having a platinum group metal ion supported thereon from an introduction passage of the packed container, passing the raw material liquid through the catalyst having a platinum group metal ion supported thereon, and discharging a reaction liquid from a discharge passage of the packed container, and
the catalyst having a platinum group metal ion supported thereon is the catalyst having a platinum group metal ion supported thereon according to any one of Claims 9 to 13.

15. The method for forming a carbon-carbon bond according to Claim 14, wherein
the formation reaction for the carbon-carbon bond is conducted in presence of an inorganic base.

16. The method for forming a carbon-carbon bond according to Claim 14 or 15, wherein
the raw material liquid (i), the raw material liquid (ii) or the raw material liquid (iii) is an inorganic base-dissolved raw material liquid prepared by dissolving raw materials and an inorganic base in water or a hydrophilic solvent, and
the formation reaction for the carbon-carbon bond is conducted by introducing the inorganic base-dissolved raw material liquid into the packed container packed with the catalyst having a platinum group metal ion supported thereon from an introduction passage of the packed container, passing the inorganic base-dissolved raw material liquid through the catalyst having a platinum group metal ion supported thereon, and discharging a reaction liquid from a discharge passage of the packed container.

17. The method for forming a carbon-carbon bond according to Claim 14 or 15, wherein
the raw material liquid (i), the raw material liquid (ii) or the raw material liquid (iii) is a hydrophobic solvent raw material liquid prepared by dissolving raw materials in a hydrophobic organic solvent, and
the formation reaction for the carbon-carbon bond is conducted by introducing a mixture of the hydrophobic solvent raw material liquid and an inorganic base aqueous solution prepared by dissolving an inorganic base into the packed container packed with the catalyst having a platinum group metal ion supported thereon from an introduction passage of the packed container, passing the hydrophobic solvent raw material liquid and the inorganic base aqueous solution through the catalyst having a platinum group metal ion supported thereon, and discharging a reaction liquid from a discharge passage of the packed container.
